# EUROPEAN PATENT APPLICATION

(11) **EP 4 600 343 A1**
(43) Date of publication of application: **13.08.2025**
(21) Application number: 23872501.4
(22) Date of filing: 28.09.2023
(51) Int. Cl.: C12N 1/20, C12N 15/01, C12N 15/31, C07K 14/32

(54) **SUPER-HIGH-MOLECULAR GAMMA-POLYGLUTAMIC ACID, BACILLUS GENUS BACTERIUM VARIANT STRAIN THAT PRODUCES SAID POLYGLUTAMIC ACID, AND METHOD FOR SCREENING FOR SAID BACILLUS GENUS BACTERIUM VARIANT STRAIN**

(30) Priority: 30.09.2022 JP 2022159018
(71) Applicant: National University Corporation Kobe University, Kobe-shi, Hyogo 657-8501 (JP)
(72) Inventor: ISHIKAWA, Shu, Kobe-shi, Hyogo 657-8501 (JP); YAMAMOTO, Junya, Kobe-shi, Hyogo 657-8501 (JP); CHUMSAKUL, Onuma, Kobe-shi, Hyogo 657-8501 (JP)
(74) Representative: Regimbeau
(86) International application number: PCT/JP2023/035341
(87) International publication number: WO 2024/071279

(57) **Abstract**

Provided is ultra-high-molecular-weight γ-polyglutamic acid with an average molecular weight of 20 million or more. Provided is a variant strain of *Bacillus* bacteria capable of producing the ultra-high-molecular-weight γ-PGA. Provided is a screening method for a variant strain of *Bacillus* bacteria capable of producing the ultra-high-molecular-weight γ-PGA. A variant strain of *Bacillus* bacteria is produced and allowed to produce γ-polyglutamic acid with an average molecular weight of 20 million or more.

## Description

### Technical Field

The present invention relates to an ultra-high-molecular-weight γ-polyglutamic acid, a variant strain of *Bacillus* bacteria that produces the polyglutamic acid, and a screening method for the variant strain of *Bacillus* bacteria.

### Background Art

Some species of *Bacillus* bacteria, including *Bacillus subtilis var. natto,* are known to be capable of producing γ-polyglutamic acid (γ-PGA). γ-PGA is γ-DL-polyglutamic acid (γ-DL-PGA) composed of L-glutamic acid and D-glutamic acid. One of the purposes for which *Bacillus* bacteria produce γ-PGA is their adaptation to external stress, such as high-salt conditions. γ-PGA is viscous, and efflux of viscous γ-PGA around cells is considered to protect cells from external stress. *Bacillus subtilis,* a representative species of *Bacillus* bacteria, is known as a model microorganism of gram-positive bacteria. Due to its safety and ease of genetic manipulation, research on *Bacillus subtilis* has been in progress.

In such *Bacillus* bacteria, γ-PGA is known to be produced by PgsBCAE, which is a γ-PGA synthetase complex composed of four proteins: PgsB, PgsC, PgsA, and PgsE. However, it has been confirmed that, among these proteins, PgsE is not essential and expression of PgsBCA is sufficient for producing γ-PGA. On the other hand, with respect to the necessity of the presence of PgsA among the PgsBCA, there are conflicting reports. For example, some reports state that PgsB, PgsC, and PgsA are all essential for γ-PGA production, whereas other reports state that PgsA is not essential. As an example of reports that state that PgsA is not essential, Patent Literature (PTL) 1 discloses that when a recombinant microorganism capable of expressing only PgsBC was produced by using a plasmid, γ-PGA was produced.

Patent Literature (PTL) 2 reports that ultra-high-molecular-weight γ-PGA with an average molecular weight of 5 million or more was obtained and that in particular, ultra-high-molecular-weight γ-PGA with an average molecular weight of 15 million was also obtained. However, in general, many reports state that γ-PGA has an average molecular weight in the range of several tens of thousands to about 5 million.

### Citation List

### Patent Literature

PTL 1: JP2009-89708A
PTL 2: JP2008-202043A

### Summary of Invention

### Technical Problem

An object is to provide ultra-high-molecular-weight γ-polyglutamic acid (γ-PGA) with an average molecular weight of 20 million or more. Another object is to provide a variant strain of *Bacillus* bacteria capable of producing ultra-high-molecular-weight γ-PGA with an average molecular weight of 20 million or more. Another object is to provide a screening method for variant strains of *Bacillus* bacteria capable of producing ultra-high-molecular-weight γ-PGA with an average molecular weight of 20 million or more.

### Solution to Problem

The present inventors, having conducted extensive research on *Bacillus* bacteria and γ-PGA, discovered that when the degrading enzyme PgdS, which has the function of cleaving the bond between D-glutamic acid and L-glutamic acid, or between L-glutamic acid and L-glutamic acid, is overexpressed in *Bacillus* bacteria that produce γ-PGA, the colonies lose their luster due to the degradation of γ-DL-PGA, but that some colonies that do not lose their luster appear. Furthermore, upon further investigation of the colonies that appeared in this manner, while some strains seemed to have formed colonies due to the loss of PgdS, other strains were also identified that appeared capable of forming colonies despite not having a deletion in PgdS. The inventors discovered that all of these strains were variants capable of producing γ-L-PGA and possessed mutations in the amino acid sequence encoding PgsA or its base sequence.

The present invention was completed through further investigation based on this finding, and the present disclosure encompasses the subject matter represented, for example, by the following.

### Item 1.

A γ-polyglutamic acid with an average molecular weight of 20 million or more.

### Item 2.

A screening method for a variant strain of *Bacillus* bacteria that produces a γ-polyglutamic acid with an average molecular weight of 20 million or more, the method comprising the following steps (I) to (III):
(I) the following step (I-1) or (I-2)
   (I-1) the step of culturing a *Bacillus* bacterium capable of expressing PgsBCA and PgdS in a medium, and
   (I-2) the step of mixing a *Bacillus* bacterium with a polynucleotide and culturing the mixture in a medium,
(II) the step of inoculating the cultured product obtained in step (I) onto a plate medium to allow colonies to form, and
(III) the step of selecting colonies with luster from the colonies formed in step (II),
wherein in step (I-2),
the *Bacillus* bacterium or the polynucleotide, or both, have a base sequence encoding a *Bacillus* bacteria-derived pgsA fragment,
the *Bacillus* bacterium or the polynucleotide, or both, have a base sequence encoding a *Bacillus* bacteria-derived pgsB fragment,
the *Bacillus* bacterium or the polynucleotide, or both, have a base sequence encoding a *Bacillus* bacteria-derived pgsC fragment, and,
the *Bacillus* bacterium or the polynucleotide, or both, have a base sequence encoding a *Bacillus* bacteria-derived PgdS fragment, and/or in step (II), colonies are allowed to form in the presence of *Bacillus* bacteria-derived PgdS protein.

### Item 3.

The screening method according to Item **2,** further comprising the step of culturing the colonies selected in step (III) and determining the average molecular weight of a γ-polyglutamic acid in the cultured product.

### Item 4.

The screening method according to Item 2 or **3,** wherein the *Bacillus* bacterium is *Bacillus subtilis.*

### Item 5.

A method for producing a γ-polyglutamic acid with an average molecular weight of 20 million or more,
the method comprising the step of culturing the variant strain of *Bacillus* bacteria obtained according to the screening method of any one of Items 2 to **4.**

### Item 6.

A variant strain of *Bacillus* bacteria that produces a γ-polyglutamic acid with an average molecular weight of 20 million or more.

### Item 7.

The variant strain of *Bacillus* bacteria according to Item **6,** wherein the variant strain of *Bacillus* bacteria is a variant strain of *Bacillus subtilis* that satisfies the following (A) and (B):
(A) the variant strain expresses PgsBCA, and
(B) PgsA consists of the amino acid sequence of any one of the following (B1) to (B4):
   (B1) the amino acid sequence from position 1 to position 56 of the amino acid sequence of SEQ ID **NO:** 1,
   (B2) an amino acid sequence in which one or more amino acids are deleted, substituted, or added in the amino acid sequence from position 1 to position 56 described in (B1),
   (B3) an amino acid sequence in which one or more amino acids are deleted, substituted, or added in the amino acid sequence from position 1 to position 368 of the amino acid sequence of SEQ ID **NO:** 1, and
   (B4) an amino acid sequence in which one or more amino acids are deleted, substituted, or added in the amino acid sequence from position 1 to position 380 of the amino acid sequence of SEQ ID **NO:** 1.

### Item 8.

The variant strain of *Bacillus* bacteria according to Item **7,** wherein the variant strain of *Bacillus* bacteria is a variant strain of *Bacillus subtilis* that satisfies at least one of the following (1) to (3):
(1) at least one mutation selected from the group consisting of the following (1-1) to (1-5) is present in the amino acid sequence from position 1 to position 56 described in (B2) to (B4): (1-1) F7S, (1-2) K20R, (1-3) V33A, (1-4) V35A, (1-5) K50R,
(2) at least one mutation selected from the group consisting of the following (2-1) to (2-98) is present in the amino acid sequence from position 57 to position 368 described in (B3) and (B4): (2-1) D66V, (2-2) G70R, (2-3) F86V, (2-4) Q87*, (2-5) M69T, (2-6) R71R, (2-7) K75K, (2-8) Q79P, (2-9) K80E, (2-10) G81E, (2-11) E90K, (2-12) F93C, (2-13) F93L, (2-14) Y98Y, (2-15) N102S, (2-16) E104K, (2-17) E104V, (2-18) E104D, (2-19) E104G, (2-20) N105Y, (2-21) P106S, (2-22) P106L, (2-23) V107I, (2-24) N112D, (2-25) D117G, (2-26) K118E, (2-27) L122P, (2-28) L122Q, (2-29) T124P, (2-30) E127G, (2-31) K133R, (2-32) L140P, (2-33) N145S, (2-34) N145D, (2-35) H146Q, (2-36) H146R, (2-37) D149Y, (2-38) D149G, (2-39) D149N, (2-40) G151S, (2-41) V152V, (2-42) V152A, (2-43) Q153R, (2-44) T158R, (2-45) A163V, (2-46) F163S, (2-47) D168N, (2-48) G171V, (2-49) G171R, (2-50) A172V, (2-51) L176L, (2-52) S177R, (2-53) A179T, (2-54) K182A, (2-55) S184P, (2-56) K187R, (2-57) A194V, (2-58) G197D, (2-59) T199A, (2-60) A208T, (2-61) G214S, (2-62) L216L, (2-63) P217L, (2-64) M226T, (2-65) H233H, (2-66) H242Y, (2-67) H242R, (2-68) H242L, (2-69) E246E, (2-70) Y247H, (2-71) D250D, (2-72) M262V, (2-73) A265V, (2-74) V271A, (2-75) H274R, (2-76) P275L, (2-77) H276R, (2-78) H276Y, (2-79) L278L, (2-80) Y284H, (2-81) Y284C, (2-82) F298L, (2-83) D299G, (2-84) D299V, (2-85) D299G, (2-86) Q300R, (2-87) W302R, (2-88) L310Q, (2-89) L315P, (2-90) K316R, (2-91) K317E, (2-92) N318I, (2-93) G321E, (2-94) F323L, (2-95) T334A, (2-96) V338A, (2-97) T347A, (2-98) D355N, wherein Q87* means that the codon encoding the amino acid residue at position 87 has become a stop codon (codon CAA encoding Q at position 87 has been substituted with TAA), and
(3) at least one mutation selected from the group consisting of the following (3-1) to (3-4) or deletion of (3-5) is present in the amino acid sequence from position 369 to position 380 described in (B4): (3-1) D370V, (3-2) H373R, (3-3) D375N, (3-4) K376E, (3-5) deletion of amino acid residues from position 369 to position 380.

### Item 9.

The γ-polyglutamic acid according to Item 1, the screening method according to any one of Items 2 to 4, the production method according to Item 5, or the variant strain of *Bacillus* bacteria according to any one of Items 6 to 8, wherein the γ-polyglutamic acid is γ-L-polyglutamic acid.

### Advantageous Effects of Invention

Ultra-high-molecular-weight γ-PGA with an average molecular weight of 20 million or more and a production method for such ultra-high-molecular-weight γ-PGA are provided. A screening method for a variant strain of *Bacillus* bacteria that produces ultra-high-molecular-weight γ-PGA with an average molecular weight of 20 million or more is provided. A variant strain of *Bacillus* bacteria that produces ultra-high-molecular-weight γ-PGA with an average molecular weight of 20 million or more is provided.

### Brief Description of Drawings

Fig. 1 shows SEQ ID NO: 1 and SEQ ID NO: 2.
Fig. 2 shows SEQ ID NO: 3 and SEQ ID NO: 4.
Fig. 3 shows SEQ ID NO: 5 and SEQ ID NO: 6.
Fig. 4 shows SEQ ID NO: 7 and SEQ ID NO: 8.
Fig. 5 shows the procedure for constructing a strain in Test Example 1.
Fig. 6 shows the procedure for constructing a strain in Test Example 1.
Fig. 7 shows the average molecular weight of γ-PGA produced by the variant strains prepared in Test Example 1.
Fig. 8 shows the procedure for constructing a DNA fragment in Test Example 2.
Fig. 9 shows the variant strains obtained in Test Example 2.

### Description of Embodiments

Embodiments that fall within the scope of the present disclosure are explained in more detail below. In the present disclosure, the term "comprising" also includes the meanings of consisting essentially of and consisting of.

### γ-polyglutamic acid with an average molecular weight of 20 million or more

The present disclosure encompasses γ-polyglutamic acid (γ-PGA) with an average molecular weight of 20 million or more. It has been generally known that the average molecular weight of γ-PGA (γ-DL-PGA) is about several hundred thousand to 3 million. As shown in PTL 2, the highest reported average molecular weight is only 15 million. The present disclosure provides γ-PGA with a very high molecular weight of 20 million or more on average.

The ultra-high-molecular-weight γ-PGA of the present disclosure can be any γ-PGA with an average molecular weight of 20 million or more, with preferable examples of the average molecular weight being 20 million to 80 million. Although not intended to limit the present disclosure, values such as 21 million, 22 million, 23 million, 24 million, 25 million, or 26 million can be examples of the lower limit. Although not intended to limit the present disclosure, values such as 70 million, 60 million, 50 million, 45 million, 40 million, 35 million, or 32 million can be examples of the upper limit. The average molecular weight can be in any of these ranges, and the average molecular weight is more preferably, for example, 20 million to 50 million, 20 million to 45 million, and so on.

Polyglutamic acid (PGA) is a type of polypeptide with glutamic acid as its polymerization unit. γ-PGA produced by *Bacillus* bacteria is a compound in which the γ-carboxyl group and the α-amino group of glutamic acid form a peptide bond, and is sometimes referred to as "poly-γ-glutamic acid." γ-PGA produced by *Bacillus* bacteria is typically in the form of γ-DL-polyglutamic acid (γ-DL-PGA), composed of L-glutamic acid and D-glutamic acid. γ-PGA composed of only L-glutamic acid can be referred to as "γ-L-polyglutamic acid" (γ-L-PGA), while γ-PGA composed of only D-glutamic acid can be referred to as "γ-D-polyglutamic acid" (γ-D-PGA). In the Test Examples described below, the molecular weight of γ-L-PGA was measured as γ-PGA. In the Test Examples, γ-L-PGA with an average molecular weight of at least 20 million was confirmed to have been produced. Additionally, as described in Test Example 1 below, the present inventors were able to obtain a group of PGAs with a very high content of γ-L-PGA relative to γ-D-PGA by using variant strains of *Bacillus* bacteria. As described below, the present disclosure provides γ-PGA with an average molecular weight of 20 million or more, in particular γ-L-PGA with an average molecular weight of 20 million or more; however, the polyglutamic acid may or may not contain γ-glutamic acid in the D-form. In other words, the γ-polyglutamic acid of the present disclosure is not limited to polymers composed exclusively of γ-L-glutamic-acid residues; it may be entirely L-form or may incorporate γ-glutamic-acid residues in the D-form. The molecular weight of γ-polyglutamic acid in the D-form also does not matter.

In the present disclosure, the average molecular weight refers to weight average molecular weight (Mw), which is measured and determined as described in the Test Examples below. Specifically, a variant strain capable of producing γ-L-PGA is inoculated on an LB agar medium and cultured at 30°C for 30 hours. Then, colonies with luster (viscosity) are collected and suspended in distilled water. The cells are removed by centrifugation, and the resulting supernatant containing γ-L-PGA is filtered through a Cosmospin Filter H (0.45 µm, Nacalai Tesque, Inc., Kyoto, Japan). The obtained filtrate is eluted with 0.1M Na₂SO₄ at 50°C at a flow rate of 1.0 mL/min, and γ-L-PGA is detected with a UV detector at a wavelength of 210 nm. The γ-PGA content is calculated by using a calibration curve prepared with food-grade PGA (Meiji Food Materia Co., Ltd., Tokyo, Japan). The molecular weight of γ-PGA is measured by using pullulan STANDARD P-82 (Showa Denko, Tokyo, Japan) as a standard sample for size exclusion chromatography. In this case, the molecular weight determination is performed using HPLC (LC-2030C Plus; Shimadzu, Kyoto, Japan) equipped with size exclusion chromatography (SEC) columns (TSKgel G6000PWXL and G4000PWXL; TOHSO, Tokyo, Japan). For the standard sample, pullulan (Shodex STANDARD P-82; Showa, Tokyo, Japan) is used, and the following substances are used: four types of γ-PGA (poly-γ-glutamic acid) with different molecular weights, whose weight average molecular weight (Mw) has been previously determined, sodium poly-L-glutamate (molecular weight: 15000 to 50000, and molecular weight: 50000 to 100000; Merck, Darmstadt, Germany), and poly-γ-glutamic acid (molecular weight: 200000 to 500000, and molecular weight: 1500000 to 2500000; Fujifilm Wako, Tokyo, Japan). For molecular weight determination, LabSolutions GPC software (Shimadzu) for HPLC is used. In this manner, weight average molecular weight (Mw) is determined by performing fractionation by chromatography and comparing the retention time with that of the molecular weight standard, such as the pullulan standard.

Moreover, as long as the average molecular weight can be determined essentially in the same manner, other plate mediums (such as minimal mediums) may be used instead of an LB agar medium; or a liquid medium may be used instead of a plate medium. When a liquid medium is used, suspension in liquid (such as distilled water) is not essential.

Although it does not limit the present disclosure, γ-PGA with an average molecular weight of 20 million or more can be easily produced by culturing the variant strains of *Bacillus* bacteria shown in the Examples described below. The culture and production method are as described below. Additionally, γ-PGA with an average molecular weight of 20 million or more can also be readily obtained by performing the screening method described below.

### A screening method for variant strains of Bacillus bacteria that produce γ-PGA with an average molecular weight of 20 million or more

The present disclosure encompasses a screening method for variant strains of *Bacillus* bacteria that produce γ-polyglutamic acid (γ-PGA) with an average molecular weight of 20 million or more, including the following steps (I) to (III):
(I) the following step (I-1) or (I-2)
   (I-1) the step of culturing a *Bacillus* bacterium capable of expressing PgsBCA and PgdS in a medium, and
   (I-2) the step of mixing a *Bacillus* bacterium with a polynucleotide and culturing the mixture in a medium,
(II) the step of inoculating the cultured product obtained in step (I) onto a plate medium to allow colonies to form, and
(III) the step of selecting colonies with luster from the colonies formed in step (II),
wherein in step (I-2),
the *Bacillus* bacterium or the polynucleotide, or both, have a base sequence encoding a *Bacillus* bacteria-derived pgsA fragment,
the *Bacillus* bacterium or the polynucleotide, or both, have a base sequence encoding a *Bacillus* bacteria-derived pgsB fragment,
the *Bacillus* bacterium or the polynucleotide, or both, have a base sequence encoding a *Bacillus* bacteria-derived pgsC fragment, and,
the *Bacillus* bacterium or the polynucleotide, or both, have a base sequence encoding a *Bacillus* bacteria-derived PgdS fragment, and/or in step (II), colonies are allowed to form in the presence of *Bacillus* bacteria-derived PgdS protein.

As mentioned above, after years of investigation, the present inventors discovered that when the degrading enzyme PgdS, which has the function of cleaving the bond between D-glutamic acid and L-glutamic acid, or between L-glutamic acid and L-glutamic acid, is overexpressed in *Bacillus* bacteria that produce γ-PGA, some colonies lose their luster due to the degradation of γ-DL-PGA, while some colonies appear without losing their luster. Furthermore, upon further investigation of the colonies, strains that seemed capable of forming colonies despite not lacking PgdS were identified in addition to strains that appeared to have formed colonies due to the absence of PgdS, as shown in the Test Examples, described below. The inventors discovered that these were variant strains capable of producing γ-L-PGA with mutations in the amino acid sequence encoding PgsA or its base sequence. Moreover, they discovered that astonishingly, ultra-high-molecular-weight γ-L-PGA with an average molecular weight of 20 million or more could be produced by using such variant strains. It is known that γ-PGA is produced due to complex PgsBCAE or PgsBCA in *Bacillus* bacteria. From these findings, they discovered that screening for variant strains of *Bacillus* bacteria that produce γ-PGA with an average molecular weight of 20 million or more can be performed using the formation of colonies that do not lose their luster even in the presence of PgdS protein as an indicator. Accordingly, the present disclosure encompasses a screening method for variant strains of *Bacillus* bacteria that produce γ-polyglutamic acid (γ-PGA) with an average molecular weight of 20 million or more, including steps (I) to (III) described above.

### Step (I)

The screening method of the present disclosure includes, as step (I), either the following step (I-1) or step (I-2):
(I-1) the step of culturing a *Bacillus* bacterium capable of expressing PgsBCA and PgdS in a medium, and
(I-2) the step of mixing a *Bacillus* bacterium with a polynucleotide and culturing the mixture in a medium.

### Step (I-1)

In step (I), the *Bacillus* bacterium capable of expressing PgsBCA and PgdS may be referred to as "parent strain 1" in the present disclosure.

Some *Bacillus* bacteria are capable of producing γ-DL-PGA, which is known to be produced by a γ-PGA synthetase complex PgsBCA, composed of three proteins: PgsB, PgsC, and PgsA. Additionally, some research reports that PgsA is not necessary for the production of γ-DL-PGA, and that γ-DL-PGA is produced by a complex PgsBC. PgdS is known to have the function of cleaving the bond between D-glutamic acid and L-glutamic acid, or between L-glutamic acid and L-glutamic acid in γ-DL-PGA. In the present disclosure, the *Bacillus* bacterium capable of expressing PgsBCA and PgdS preferably can express PgsBCA and PgdS in a medium commonly used in culturing *Bacillus* bacteria capable of producing

### γ-DL-PGA.

Although not limiting the present disclosure, examples of such a culture medium include an LB agar medium (containing tryptone, yeast extract, sodium chloride, and agar, pH: 7). Additionally, although not limiting the present disclosure, examples of culture conditions include 30 to 37°C for 12 to 24 hours. Culture can be performed according to a known culture procedure, regardless of whether under either light or dark conditions and whether it is static or shaking culture depending on the type of medium. In this manner, the expression and its confirmation can be performed.

The *Bacillus* bacterium can be any *Bacillus* bacterium capable of expressing γ-DL-PGA; although not limiting the present disclosure, examples include *Bacillus subtilis,* such as *natto* bacteria *(Bacillus subtilis var. natto), Bacillus amyloliquefaciens, Bacillus licheniformis,* and *Bacillus megaterium.* The *Bacillus* bacterium can be, for example, preferably *Bacillus subtilis* and *Bacillus amyloliquefaciens,* with *Bacillus subtilis* being more preferable.

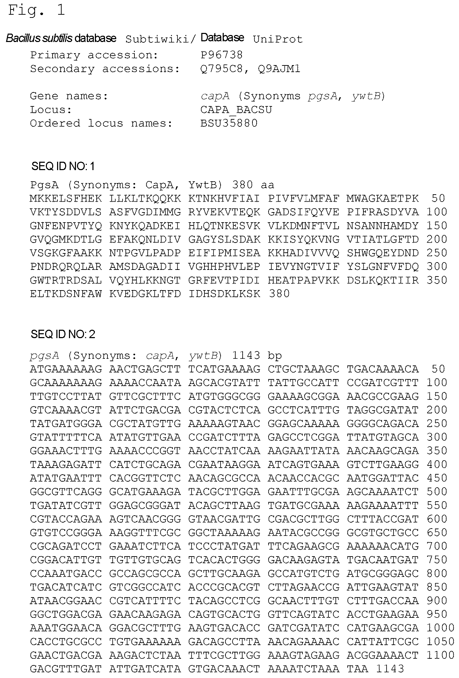

In parent strain 1, the sequences of PgsA, PgsB, PgsC, and PgdS are not restricted. While not limiting the present disclosure, an example of amino acid sequences encoding PgsA of *Bacillus subtilis,* which is a species of *Bacillus* bacteria, is the amino acid sequence of SEQ ID NO: 1. PgsA may also be referred to as "PgsAA," "CapA," or "YwtB." An example of base sequences encoding the amino acid sequence of SEQ ID NO: 1 is the base sequence of SEQ ID NO: 2. The pgsA gene may also be referred to as a "pgsAA," "capA," or "ywtB" gene. Fig. 1 shows these sequences.

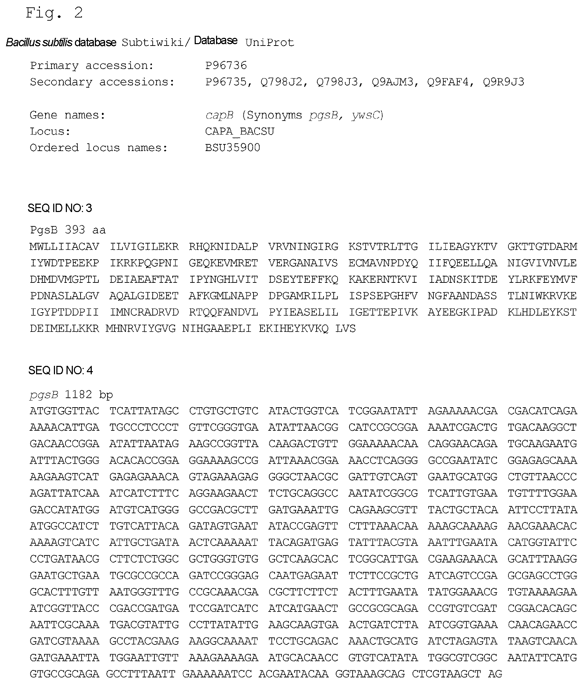

Additionally, an example of the amino acid sequence encoding PgsB of *Bacillus subtilis* is the amino acid sequence of SEQ ID NO: 3, and an example of the base sequence encoding the amino acid sequence of SEQ ID NO: 3 is the base sequence of SEQ ID NO: 4. Fig. 2 shows these sequences.

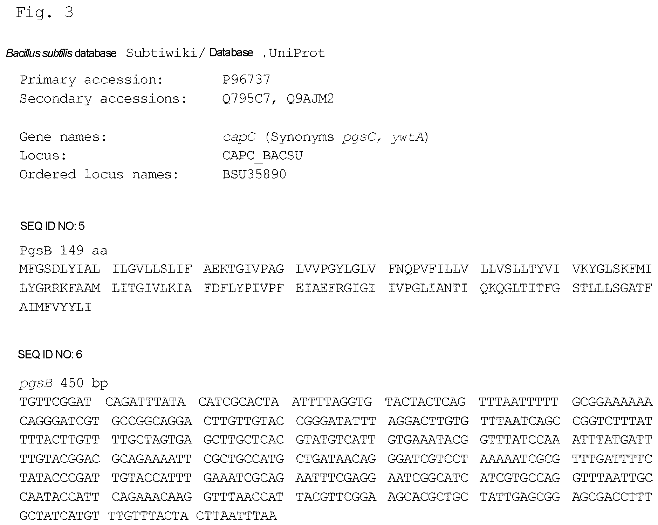

Additionally, an example of the amino acid sequence encoding PgsC of *Bacillus subtilis* is the amino acid sequence of SEQ ID NO: 5, and an example of the base sequence encoding the amino acid sequence of SEQ ID NO: 5 is the base sequence of SEQ ID NO: 6. Fig. 3 shows these sequences.

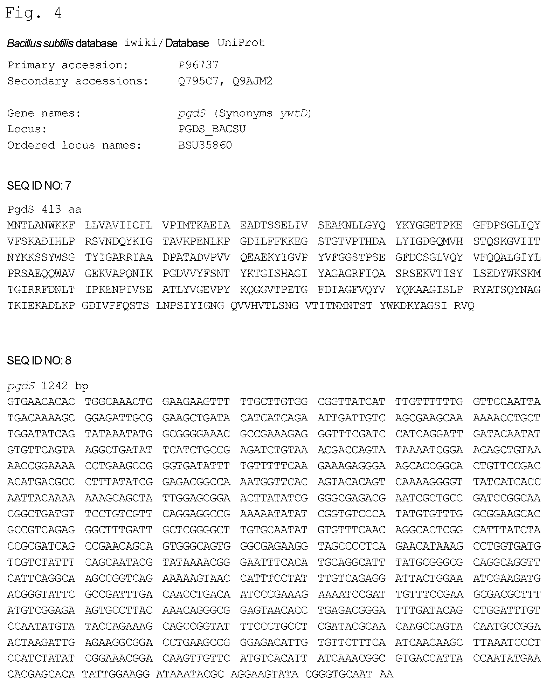

Additionally, an example of the amino acid sequence encoding PgdS of *Bacillus subtilis* is the amino acid sequence of SEQ ID NO: 7, and an example of the base sequence encoding the amino acid sequence of SEQ ID NO: 7 is the base sequence of SEQ ID NO: 8. Fig. 4 shows these sequences.

Additionally, as long as the parent strain is capable of expressing PgsBCA and PgdS, the proteins PgsA, PgsB, PgsC, and PgdS may each have modifications in the sequences described above.

An example of PgsA with such modification is an amino acid sequence having a sequence identity of at least 95%, preferably at least 98%, more preferably at least 99%, or at least 99.5% with the amino acid sequence of SEQ ID NO: 1. An example of PgsB with such modification is an amino acid sequence having a sequence identity of at least 95%, preferably at least 98%, more preferably at least 99%, or at least 99.5% with the amino acid sequence of SEQ ID NO: 3. An example of PgsC with such modification is an amino acid sequence having a sequence identity of at least 95%, preferably at least 98%, more preferably at least 99%, or at least 99.5% with the amino acid sequence of SEQ ID NO: 5. An example of PgdS with such modification is an amino acid sequence having a sequence identity of at least 95%, preferably at least 98%, more preferably at least 99%, or at least 99.5% with the amino acid sequence of SEQ ID NO: 7.

Furthermore, an example of PgsA with such modification is an amino acid sequence with one or more amino acids deleted, substituted, or added in the amino acid sequence of SEQ ID NO: 1. An example of PgsB with such modification is an amino acid sequence with one or more amino acids deleted, substituted, or added in the amino acid sequence of SEQ ID NO: 3. An example of PgsC with such modification is an amino acid sequence with one or more amino acids deleted, substituted, or added in the amino acid sequence of SEQ ID NO: 5. An example of PgdS with such modification is an amino acid sequence with one or more amino acids deleted, substituted, or added in the amino acid sequence of SEQ ID NO: 7. The phrase "one or more" in these individual sequences means, for example, 1 to 30, preferably 1 to 25, 1 to 20, 1 to 15, 1 to 10, 1 to 8, 1 to 5, 1 to 4, 1 to 3, or 1 or 2.

As stated above, since parent strain 1 is capable of expressing complex PgsBCA and PgdS, each modification is limited to modification that allows the expression of PgsBCA and PgdS. From this perspective, preferable examples of modifications include those resulting in conservative substitution of amino acids as compared to each of the amino acid sequences of SEQ ID NOs: 1, 3, 5, and 7.

In the present disclosure, the identity of amino acid sequences refers to the degree to which two or more contrastable amino acid sequences match each other. The level of amino acid sequence identity can be determined, for example, by using FASTA (sequence analysis tool) with default parameters. The level of amino acid sequence identity can also be determined by using the BLAST algorithm by Karlin and Altschul (Karlin S, Altschul SF., Methods for assessing the statistical significance of molecular sequence features by using general scorings schemes, Proc Natl Acad Sci USA. 87: 2264-2268(1990); Karlin S, Altschul SF., Applications and statistics for multiple high-scoring segments in molecular sequences, Proc Natl Acad Sci USA. 90: 5873-7(1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/).

In the present disclosure, conservative substitution refers to substitution of an amino acid residue with another amino acid residue having a side chain of similar nature. For example, conservative substitution means substitution between amino acid residues with basic side chains, such as arginine, lysine, or histidine. Additionally, other examples of conservative substitution include substitutions between amino acid residues having an acidic side chain, such as aspartic acid or glutamic acid; substitution between amino acid residues having an uncharged polar side chain, such as glycine, asparagine, glutamine, serine, threonine, tyrosine, or cysteine; substitution between amino acid residues having a non-polar side chain, such as alanine, valine, leucine, isoleucine, proline, phenylalanine, methionine, or tryptophan; substitution between amino acid residues having a β-branched side chain, such as threonine, valine, or isoleucine; and substitution between amino acid residues having an aromatic side chain, such as tyrosine, phenylalanine, tryptophan, or histidine.

The base sequence of SEQ ID NO: 2 may be modified within the range of modification to the amino acid sequence of SEQ ID NO: 1. The base sequence of SEQ ID NO: 4 may be modified within the range of modification to the amino acid sequence of SEQ ID NO: 3. The base sequence of SEQ ID NO: 6 may be modified within the range of modification to the amino acid sequence of SEQ ID NO: 5. The base sequence of SEQ ID NO: 8 may be modified within the range of modification to the amino acid sequence of SEQ ID NO: 7.

Without limiting the present disclosure, for example, for *Bacillus amyloliquefaciens* (genome sequence: accession number CP002634 *(Bacillus amyloliquefaciens* LL3)), which is a species of *Bacillus* bacteria, an example of the amino acid sequence encoding PgsA is the amino acid sequence of SEQ ID NO: 9. An example of the amino acid sequence encoding PgsB is the amino acid sequence of SEQ ID NO: 10, which is the amino acid sequence indicated by accession number AEB65253 in the GenBank database (393 amino acid residues, last updated on January 30, 2014). An example of the amino acid sequence encoding PgsC is the amino acid sequence of SEQ ID NO: 11, which is the amino acid sequence indicated by accession number AEB65252 in the GenBank database (149 amino acid residues, last updated on January 30, 2014). An example of the amino acid sequence encoding PgdS of *Bacillus amyloliquefaciens* LL3 is the amino acid sequence of SEQ ID NO: 12, which is the amino acid sequence indicated by accession number AEB65249 in the GenBank database (411 amino acid residues, last updated on January 30, 2014).

*Bacillus* bacteria other than *Bacillus subtilis* may also have modifications in their sequences, and these modifications are explained in the same manner as described above.

As described above, it has been known that *Bacillus* bacteria produce γ-DL-PGA with the γ-PGA synthetase complex PgsBCAE. From this perspective, PgsBCA that can be expressed by parent strain 1 may further include PgsE. In other words, the PgsBCA expressed by parent strain 1 need to include at least three proteins, i.e., PgsB, PgsC, and PgsA, and may also be a complex that includes PgsE. Thus, *Bacillus* bacteria capable of expressing PgsBCA and PgdS include *Bacillus* bacteria capable of expressing PgsBCAE and also capable of expressing PgdS. The amino acid sequences encoding PgsE of *Bacillus* bacteria are also known; an example of the amino acid sequence encoding PgsE of *Bacillus subtilis* is the amino acid sequence of SEQ ID NO: 13, and an example of the base sequence encoding the amino acid sequence of SEQ ID NO: 13 is the base sequence of SEQ ID NO: 14. Similarly, the sequence of SEQ ID NO: 13 may also have modifications as described above. Examples include amino acid sequences having a sequence identity of at least 95%, preferably at least 98%, more preferably at least 99%, or at least 99.5% with the amino acid sequence of SEQ ID NO: 13. Additionally, examples also include amino acid sequences in which one or more amino acids are deleted, substituted, or added in the amino acid sequence of SEQ ID NO: 13. These mutations are explained in the same manner as described above.

In step (I-1), parent strain 1 is cultured in a medium. Although not limiting the present disclosure, examples of culture mediums include, but are not limited to, mediums commonly used in culturing the *Bacillus* bacteria capable of producing γ-DL-PGA mentioned above. The medium may be the same as that in step (I-2), described below. The medium can be either a liquid medium or a plate medium, for example, with a liquid medium being preferable. Additionally, the inoculation amount of parent strain 1 into a medium is not specified, and the culture can be performed according to a known culture procedure. Culture conditions can be, for example, 30 to 37°C for 2 to 5 hours. Culture can be performed regardless of whether under light or dark conditions and whether it is either static or shaking culture.

The medium may contain a transforming substance that induces mutation. Examples of transforming substances include DNA fragments. The concentration of the transforming substance in the medium is not limited and can be determined as appropriate. Additionally, stimuli, such as light or heat, may be applied to parent strain 1 during culture in the medium. These substances and stimuli can be used alone or in a combination of two or more. Additionally, parent strain 1 and/or such transforming substances may contain any optional genes, such as genetic markers, and any optional sequences, such as promoters. Such optional genes and sequences can be explained in the same manner as in step (I-2), described below. The culture may be repeated **(e.g.,** subculture or culture performed two or more times in the presence of a transforming substance).

The culture described above is performed with the aim of inducing transformation in parent strain 1 through natural mutations or other means to make it easier to obtain the transformant (variant strain) of parent strain 1. As described below, numerous mutations were observed in the amino acid sequence encoding the PgsA protein or its base sequence of the variant strains of *Bacillus* bacteria that produce γ-PGA with an average molecular weight of 20 million or more. From this perspective, the culture described above is preferably performed with the aim of inducing transformation of a strain into a variant strain that has mutations in the amino acid sequence encoding the PgsA protein or its base sequence. The cultured product obtained by culture in this manner can be used in the subsequent step, step (II).

### Step (I-2)

The screening method of the present disclosure includes the step of mixing a *Bacillus* bacterium with a polynucleotide and culturing the mixture in a medium as step (I-2). In step (I-2), the *Bacillus* bacterium or the polynucleotide, or both, have a base sequence encoding a *Bacillus* bacteria-derived pgsA fragment, the *Bacillus* bacterium or the polynucleotide, or both, have a base sequence encoding a *Bacillus* bacteria-derived pgsB fragment, and the *Bacillus* bacterium or the polynucleotide, or both, have a base sequence encoding a *Bacillus* bacteria-derived pgsC fragment. The *Bacillus* bacterium mixed with the polynucleotide in step (I-2) may be referred to as "parent strain 2."

While not limiting the present disclosure, preferable examples of parent strain 2 include known *Bacillus* bacteria capable of producing γ-DL-PGA. As mentioned above, examples of *Bacillus* bacteria capable of producing γ-DL-PGA include *Bacillus subtilis* (e.g., *Bacillus subtili natto)* and *Bacillus amyloliquefaciens.* Preferable examples of *Bacillus* bacteria include *Bacillus subtilis* and *Bacillus amyloliquefaciens,* with *Bacillus subtilis* being more preferable. These known *Bacillus* bacteria capable of producing γ-DL-PGA typically carry pgsBCAE gene and pgdS gene.

Parent strain 2 for use may be, for example, any of the following strains: a strain with the pgsA gene deleted, a strain with the pgsB gene deleted, or a strain with the pgsC gene deleted from such *Bacillus* bacteria. Parent strain 2 may have two or more deficiencies of these genes. Furthermore, parent strain 2 may also be a parent strain prepared by first deleting the pgsA gene by using a genetic engineering technique and then introducing a base sequence encoding a *Bacillus* bacteria-derived pgsA fragment. The same explanation applies to the pgsB and pgsC genes.

Parent strain 2 or the polynucleotide, or both, have a base sequence encoding a *Bacillus* bacteria-derived pgsA fragment. Parent strain 2 and the polynucleotide may both have a base sequence encoding such a fragment; preferably only either parent strain 2 or the polynucleotide has a base sequence encoding such a fragment.

Similarly, parent strain 2 or the polynucleotide, or both, have a base sequence encoding a *Bacillus* bacteria-derived pgsB fragment. Parent strain 2 and the polynucleotide may both have a base sequence encoding such a fragment; preferably only either parent strain 2 or the polynucleotide has a base sequence encoding such a fragment.

Similarly, parent strain 2 or the polynucleotide, or both, have a base sequence encoding a *Bacillus* bacteria-derived pgsC fragment. Parent strain 2 and the polynucleotide may both have a base sequence encoding such a fragment; preferably only either parent strain 2 or the polynucleotide has a base sequence encoding such a fragment.

In the present disclosure, the pgsA gene possessed by *Bacillus* bacteria that has not been modified by a genetic engineering technique (parent strain 2) is also described as a base sequence encoding a *Bacillus* bacteria-derived pgsA fragment. The same explanation applies to other genes such as the pgsB gene.

For example, in one embodiment of the present disclosure, when a strain with deficiency of the pgsA gene is used as parent strain 2, the polynucleotide contains a base sequence encoding a *Bacillus* bacteria-derived pgsA fragment. When a strain with deficiency of the pgsB gene is used as parent strain 2, the polynucleotide contains a base sequence encoding a *Bacillus* bacteria-derived pgsB fragment. When a strain with deficiency of the pgsBC gene is used as parent strain 2, the polynucleotide contains a base sequence encoding a *Bacillus* bacteria-derived pgsB fragment and a base sequence encoding a *Bacillus* bacteria-derived pgsC fragment. When a strain with deficiency of the pgsBCA gene is used as parent strain 2, the polynucleotide contains a base sequence encoding a *Bacillus* bacteria-derived pgsB fragment, a base sequence encoding a *Bacillus* bacteria-derived pgsC fragment, and a base sequence encoding a *Bacillus* bacteria-derived pgsA fragment.

*Bacillus* bacteria capable of producing γ-DL-PGA typically contain the pgdS gene. Thus, parent strain 2 may have the pgdS gene or may be deficient in the pgdS gene. The polynucleotide may or may not contain a base sequence encoding a *Bacillus* bacteria-derived pgdS fragment. When parent strain 2 deficient in the pgdS gene is used, the polynucleotide preferably contains a base sequence encoding a *Bacillus* bacteria-derived pgdS fragment. Additionally, when colonies are to be formed in the presence of *Bacillus* bacteria-derived PgdS protein in step (II), described below, parent strain 2 and the polynucleotide in step (I-2) do not necessarily need to possess the pgdS gene (a base sequence encoding a PgdS fragment).

*Bacillus* bacteria capable of producing γ-DL-PGA typically contain pgsE gene. Thus, parent strain 2 may have the pgsE gene or may be deficient in the pgsE gene. When parent strain 2 having the pgsE gene is used, the polynucleotide may or may not contain a base sequence encoding a *Bacillus* bacteria-derived pgsE fragment. When parent strain 2 deficient in the pgsE gene is used, the polynucleotide may or may not contain a base sequence encoding the pgsE fragment; however, the polynucleotide preferably contains a base sequence encoding the pgsE fragment.

The deletion of the genes described above in *Bacillus* bacteria capable of producing γ-DL-PGA can be performed according to a known genetic engineering technique. The polynucleotide described above can also be created according to a known genetic engineering technique.

The base sequences encoding the genes (fragments) of pgsA, pgsB, pgsC, pgdS, and pgsE in parent strain 2 and the polynucleotide are not limited as long as they are derived from *Bacillus* bacteria. While not limiting the present disclosure, examples of such sequences include base sequences encoding the amino acid sequences of SEQ ID NO: 1, 3, 5, 7, and 13, and examples of the base sequences include the base sequences of SEQ ID NO: 2, 4, 6, 8, and 14. Furthermore, while not limiting the present disclosure, examples of base sequences encoding the genes (fragments) of pgsA, pgsB, pgsC, and pgdS include the base sequences encoding the amino acid sequences of SEQ ID NO: 9, 10, 11, and 12.

The base sequences encoding the gene (fragment) of pgsA, pgsB, pgsC, pgdS, or pgsE may have modification in the sequences.

The screening method of the present disclosure is performed with the aim of inducing transformation in parent strain 2 to make it easier to obtain a transformant (variant strain) of parent strain 2 by mixing parent strain 2 with a polynucleotide and culturing the mixture in step (I-2). The subsequent step, step (II), is of selecting colonies with luster in the presence of PgdS protein from among the variant strains obtained through step (I-2). Thus, from the viewpoint of simply obtaining a transformant in step (I-2), the base sequences encoding the individual genes (fragments) in parent strain 2 and the polynucleotide may have any modifications in the sequences. Thus, step (I-2), like step (I-1), is performed with the aim of inducing transformation to make it easier to obtain a transformant (variant strain) of the parent strain.

Although there is no limitation, an example of modification is a base sequence in which one or more bases are deleted, substituted, or added in base sequence of SEQ ID NO: 2, from the viewpoint of more efficiently screening for variant strains of *Bacillus* bacteria that produce γ-PGA with an average molecular weight of 20 million or more. An example of such modification is a base sequence in which one or more bases are deleted, substituted, or added in base sequence of SEQ ID NO: 4. An example of such modification is a base sequence in which one or more bases are deleted, substituted, or added in base sequence of SEQ ID NO: 6. An example of such modification is a base sequence in which one or more bases are deleted, substituted, or added in base sequence of SEQ ID NO: 8. An example of such modification is a base sequence in which one or more bases are deleted, substituted, or added in base sequence of SEQ ID NO: 14. In these cases, "one or more" can be, independently, any of 1 to 30, 1 to 20, 1 to 15, 1 to 10, 1 to 5, 1 to 4, 1 to 3, 1, or 2.

From this viewpoint, a modified base sequence hybridizes under stringent conditions with a polynucleotide composed of a DNA sequence complementary to the individual base sequence (DNA sequence) before modification. The phrase "hybridizes under stringent conditions" means that two polynucleotides can hybridize with each other under standard hybridization conditions, which are described by Sambrook et al. in Molecular Cloning: A laboratory manual (1989), Cold Spring Harbor Laboratory Press, New York, USA. More specifically, "stringent conditions" means performing hybridization in 6.0x SSC at about 45°C and then washing with 2.0x SSC at 50°C.

As described below, the variant strains of *Bacillus* bacteria that produce γ-PGA with an average molecular weight of 20 million or more were found to have numerous mutations in their amino acid sequence encoding the PgsA protein or the base sequences. For example, as can be understood from Test Example 1, described below, even the deletion of the amino acids from position 57 to position 380 from the amino acid sequence of SEQ ID NO: 1 (Example 1) gave a variant capable of producing ultra-high-molecular-weight γ-PGA with an average molecular weight of 20 million or more. From this, an example of such modification can be a base sequence in which any number of bases within the range of 1 to 972 are deleted from the base sequence of SEQ ID NO: 2. Furthermore, as can be understood from the Test Examples described below, for example, even the deletion of multiple amino acids in Examples 2 to 5 gave variants that produced ultra-high-molecular-weight γ-PGA with an average molecular weight of 20 million or more. This indicates that modification can be appropriately made in consideration of the Test Examples described below. Additionally, this also indicates that the base sequence encoding the pgsA fragment may have any modification in SEQ ID NO: 2 in the screening method of the present disclosure.

Among the five base sequences individually encoding pgsA, pgsB, pgsC, pgdS, and pgsE, either only one sequence may have modification, or two or more sequences may have modification. As described above, since numerous mutations were observed in the amino acid sequence encoding PgsA in variant strains of *Bacillus* bacteria that produce γ-PGA with an average molecular weight of 20 million or more, using a parent strain and/or polynucleotide having modification in at least the base sequence encoding pgsA is preferably mentioned.

As described above, variant strains of *Bacillus* bacteria that produce γ-PGA typically produce γ-PGA through a complex PgsBCA, which is composed of collaborating three proteins: PgsB, PgsC, and PgsA (or complex PgsBCAE composed of collaborating four proteins). Thus, from the viewpoint of more efficiently screening for variant strains of *Bacillus* bacteria that produce γ-PGA with an average molecular weight of 20 million or more, the modified base sequences have modifications that enable the individual amino acid sequences coded by the modified base sequences to constitute the corresponding proteins of PgsBCA (or PgsBCAE) that is useful as a γ-PGA synthetase complex.

Examples of amino acid sequences that encode PgsA, PgsB, PgsC, or PgsE, and that are encoded by the base sequences contained in parent strain 2 and/or the polynucleotide, include, but are not limited to, the amino acid sequence of SEQ ID NO: 1, the amino acid sequence of SEQ ID NO: 3, the amino acid sequence of SEQ ID NO: 5, and the amino acid sequence of SEQ ID NO: 13 respectively, and these sequences may also have modifications. Additionally, examples of amino acid sequences that encode PgdS, and that are encoded by the base sequences described above, which are usable for the parent strain and/or polynucleotide, include the amino acid sequence of SEQ ID NO: 7, which may also have modifications.

The modification is explained in the same manner as above. Specifically, for PgsA, examples include amino acid sequences with at least 95%, preferably at least 98%, more preferably at least 99%, or at least 99.5% sequence identity with the amino acid sequence of SEQ ID NO: 1. Additionally, examples also include amino acid sequences in which one or more amino acids are deleted, substituted, or added in the amino acid sequence of SEQ ID NO: 1. These mutations are explained in the same manner as described above. The same explanation also applies to PgsB, PgsC, PgsE, and PgdS.

Furthermore, from the viewpoint of more efficiently screening for variant strains of *Bacillus* bacteria that produce γ-PGA with an average molecular weight of 20 million or more in the present disclosure, preferable variant strains are capable of expressing the complex PgsBCA (or PgsBCAE) composed of collaborating PgsB, PgsC, and PgsA (and additionally PgsE). Thus, examples of favorable modifications include modifications resulting in conservative amino acid substitutions as compared to the amino acid sequences of SEQ ID NO: 1, 3, 5, and/or 13.

Additionally, from a similar perspective, it is preferred that the base sequence encoding the pgdS fragment usable in parent strain 2 and/or the polynucleotide also has the functionality of cleaving the bond between D-glutamic acid and L-glutamic acid, or between L-glutamic acid and L-glutamic acid, as conventionally known for PgdS protein. From this perspective, examples of preferable modifications to PgdS include those that result in conservative amino acid substitution as compared to the amino acid sequence of SEQ ID NO: 7.

In these cases, amino acid sequence identity is explained in the same manner as described above, and conservative substitution is also explained in the same manner as described above.

The base sequence of SEQ ID NO: 2 may be modified within the scope of modifications to the amino acid sequence of SEQ ID NO: 1. The base sequence of SEQ ID NO: 4 may be modified within the scope of modifications to the amino acid sequence of SEQ ID NO: 3. The base sequence of SEQ ID NO: 6 may be modified within the scope of modifications to the amino acid sequence of SEQ ID NO: 5. The base sequence of SEQ ID NO: 8 may be modified within the scope of modifications to the amino acid sequence of SEQ ID NO: 7. The base sequence of SEQ ID NO: 14 may be modified within the scope of modifications to the amino acid sequence of SEQ ID NO: 13.

However, as described above, the variant strains of *Bacillus* bacteria that produce γ-PGA with an average molecular weight of 20 million or more were found to have numerous mutations in the amino acid sequence encoding the PgsA protein or the base sequence. For example, as can be understood from Test Example 1 described below, even the deletion of the amino acids at position 57 to position 380 from the amino acid sequence of SEQ ID NO: 1 (Example 1) gave a variant capable of producing ultra-high-molecular-weight γ-PGA with an average molecular weight of 20 million or more. Thus, an example of such modification can be a sequence in which any number of amino acids within the range 1 to 324 are deleted from the amino acid sequence of SEQ ID NO: 1, in other words, a base sequence in which any number of bases within the range of 1 to 972 are deleted from the base sequence of SEQ ID NO: 2. As can be understood from the Test Examples described below, variants that produce ultra-high-molecular-weight γ-PGA with an average molecular weight of 20 million or more were obtained, for example, when multiple amino acids were deleted in Examples 2 to 5. This also indicates that such modifications can be appropriately made in consideration of the Test Examples described below. Thus, the base sequence encoding a pgsA fragment may have any modification in SEQ ID NO: 2 in the screening method of the present disclosure.

The amino acid sequences encoding the proteins PgsA, PgsB, PgsC, PgdS, and PgsE and their corresponding base sequences are also known for *Bacillus* bacteria other than *Bacillus subtilis,* and can be explained in the same manner. Additionally, *Bacillus* bacteria other than *Bacillus subtilis* may also have modifications in their sequences, and these modifications are explained in the same manner as described above.

Parent strain 2 and/or the polynucleotide may further contain any optional genes, such as genetic markers, and any optional sequences, such as promoters, as long as the effects of the present disclosure are achieved.

Examples of genetic markers as optional genes include drug-resistant genes, such as chloramphenicol resistance gene (cat), kanamycin resistance gene (kan), erythromycin resistance gene (erm), and spectinomycin resistance gene (spec), and auxotrophic markers. Examples of optional genes further include α-amylase gene (amyE), alkaline protease gene (aprE), rpsI gene, and tufA gene. These genes and their base sequences are known. These can be used alone or in a combination of two or more, and can be located in any position.

Although not limiting the present disclosure, examples of preferred parent strain 2 include the strains described in the Test Examples below. However, the present disclosure is not limited to the strains. Additionally, parent strain 2 may be a strain that has undergone some genetic manipulation, which can be performed by using known methods, such as natural transformation, plasmid-based techniques, or CRISPR-Cas. For example, parent strain 2 may be a recombinant strain that expresses the PgdS protein at a high level (overexpressing PgdS). For instance, a strain that expresses the PgdS protein at a high level can be obtained according to a method of cloning the pgdS gene into a plasmid in an expressible form and introducing the plasmid, or a method of replacing the original pgdS promoter with a promoter that is constitutively (constantly) activated at a high level. Typically, strains that produce γ-DL-PGA form shiny colonies resembling water droplets. However, strains that express PgdS at high levels lose this luster, and furthermore, the colony morphology becomes flattened. These characteristics can serve as one of the indicators that the parent strain is a strain that expresses PgdS at high levels.

Although not limiting the present disclosure, examples of preferred polynucleotides include those containing a base sequence in which a base sequence encoding a pgsB fragment, a base sequence encoding a pgsC fragment, a base sequence encoding a pgsA fragment, and a base sequence encoding a pgsE fragment of *Bacillus* bacteria are linked (which may be referred to as a "pgsBCAE base sequence" below).

Each of the linkages between these base sequences may be independently a direct linkage, a linkage via a linker, or a linkage via a base sequence encoding any gene different from these base sequences (e.g., rpsI gene). Any linker and/or gene can be used as long as they do not interfere with the effects of the present disclosure.

Although not limiting the present disclosure, examples of preferable polynucleotides include those in which a base sequence encoding a pgsB fragment, a base sequence encoding a pgsC fragment, a base sequence encoding a pgsA fragment, and a base sequence encoding a PgsE fragment of *Bacillus* bacteria are sequentially linked from the 5' end to the 3' end.

Additionally, the polynucleotide may not contain a base sequence encoding a pgsE fragment. The polynucleotide may also not contain a base sequence encoding a pgsB fragment. The polynucleotide may not contain a base sequence encoding a pgsC fragment. The polynucleotide may not contain a base sequence encoding a pgsA fragment. While the combination of these fragments in the polynucleotide is not limited, numerous variant strains with mutations in PgsA were confirmed among variant strains of *Bacillus* bacteria that produce γ-L-PGA with an average molecular weight of 20 million or more as described below. Thus, from the viewpoint of more efficiently screening for variant strains of *Bacillus* bacteria that produce γ-L-PGA with an average molecular weight of 20 million or more, it is preferred that the polynucleotide contains at least a base sequence encoding a pgsA fragment. Additionally, these may also contain a base sequence encoding a pgdS fragment of *Bacillus* bacteria.

For example, the polynucleotide preferably further contains a base sequence encoding a genetic marker. Examples of genetic markers include drug-resistant genes, such as chloramphenicol resistance gene (cat) described above, and auxotrophic markers, as mentioned above. These may be used alone or in a combination of two or more. The genetic marker can be located at any position within the polynucleotide.

While not limiting the present disclosure, the polynucleotide may be chromosomal DNA of a variant strain or chromosomal DNA of a strain having a mutation adjacent to a genetic marker, either directly or via any linker. For example, when the polynucleotide is synthesized according to a method such as PCR, there is no size limitation for the polynucleotide. The number of bases in the homologous recombination region is, for example, 0.2 to 3 kbp, preferably 0.2 to 1.5 kbp, and more preferably 1.5 to 3 kbp.

While not limiting the present disclosure, preferred examples of parent strain 2 and the polynucleotides include the strains described in the Test Examples described below. However, the present disclosure is not limited to those strains.

In step (I-2), as described above, a *Bacillus* bacterium and a polynucleotide are mixed and cultured in a medium. At the time of mixing, the polynucleotide may be a DNA fragment (e.g., a PCR product) or a polynucleotide incorporated into a vector (plasmid), and their form is not limited. Preferably, the polynucleotide is, for example, a DNA fragment. The polynucleotide may be referred to as a "DNA fragment etc." below.

There are no restrictions on the means of mixing a parent strain with a DNA fragment etc. However, for simplicity, it is sufficient to mix a parent strain with a DNA fragment etc. in a liquid medium.

The medium used in step (I-2) is not limited as long as it allows for the growth of *Bacillus* bacteria, and can be either a liquid medium or a plate medium, with a liquid medium being preferred. While not limiting the present disclosure, examples of liquid mediums include C-I medium and C-II medium. Mediums such as C-I medium and C-II medium are known in the art. Examples of their compositions are described in the Test Examples below.

The amount of parent strain 2 and DNA fragment etc. mixed is not limited as long as the effects of the present disclosure are achieved, and the amount can be determined as appropriate. For example, parent strain 2 preferably in an amount of about 1×10⁶ to 1×10⁹ cells/mL, more preferably about 5 to 10×10⁸ cells/mL, can be mixed with a DNA fragment (e.g., about 0.1 to 1 µg/mL) in a liquid medium. For a solid medium, the amount can be determined with reference to this amount.

In this manner, after parent strain 2 is mixed with a DNA fragment etc., the parent strain is cultured in a medium in the presence of the DNA fragment etc. Preferably, after parent strain 2 is made into competent cells in the medium, the cells are mixed with a DNA fragment etc., and then the parent strain is cultured in a medium in the presence of the DNA fragment etc. While the culture conditions are not limited as long as parent strain 2 can be cultured, preferable culture conditions are, for example, 30 to 37°C for 2 to 8 hours, more preferably 2 to 5 hours. Culture can be performed under light or dark conditions, and either as static culture or shaking culture. For example, because *Bacillus* bacteria have the nature to take up foreign DNA through natural transformation, such culture allows for the transformation of the parent strain.

While not limiting the present disclosure, the transformation may use the principle of homologous recombination during genome modification of *Bacillus subtilis.* For example, when a base sequence encoding any of the genes mentioned above (e.g., a pgsA fragment) is inserted into amyE, the 5' region of amyE, a promoter region, the base sequence, a gene marker, and the 3' region of amyE can be individually amplified by PCR, then DNA fragments are linked by overextension PCR (recombinant PCR), Gibson assembly, etc., or these sequences can be partially synthesized using artificial synthetic genes and then linked, or even all DNA fragments can be synthesized. Introducing this into parent strain 2 using the natural transformation ability would result in the introduction of the amyE 5', promoter, base sequence, gene marker, and amyE 3' near the middle of amyE. The insertion of pgdS downstream of tufA in the Test Examples described below also used a similar principle. As noted above, the method of obtaining the polynucleotide is not limited and can be performed according to a known technique in the art. For example, the polynucleotide can be obtained by using PCR. Primers and other elements can be appropriately determined according to the sequence of the desired polynucleotide. Preferable examples of primers include those shown in the Test Examples described below.

The origin of parent strain 2 and the polynucleotide (more specifically, the origin of the base sequence encoding any of the genes described above **(e.g.,** a pgsA fragment) that constitutes the polynucleotide) is not limited as long as parent strain 2 and the polynucleotide are both from *Bacillus* bacteria. Examples of *Bacillus* bacteria, as mentioned above, include *Bacillus subtilis, Bacillus amyloliquefaciens,* and *Bacillus licheniformis.* For example, parent strain 2 may be *Bacillus subtilis,* and the polynucleotide may be derived from a *Bacillus* bacterium other than *Bacillus subtilis* **(e.g.,** derived from *Bacillus amyloliquefaciens).* Parent strain 2 may be a *Bacillus* bacterium other than *Bacillus subtilis,* and the polynucleotide may be derived from *Bacillus subtilis.* Parent strain 2 and the polynucleotide both may be derived from *Bacillus subtilis.* Parent strain 2 and the polynucleotide both may be derived from a *Bacillus* bacterium other than *Bacillus subtilis.* Preferably, parent strain 2 and the polynucleotide are (derived from) the same species; more preferably, both are (derived from) *Bacillus subtilis.* The polynucleotide may be used alone or in combination of two or more types.

In this manner, a *Bacillus* bacterium (parent strain 2) is mixed with a polynucleotide and cultured in a medium in step (I-2), and transformation is thus induced in the parent strain. This step is preferably performed with the aim of inducing a parent strain to transform into a recombinant organism (variant strain) that has mutations in the amino acid sequence encoding the PgsA protein or the base sequence. The thus-obtained cultured product can be used in the subsequent step, step (II).

### Step (II)

In step (II), the cultured product obtained by the culturing in step (I) is inoculated onto a plate medium to form colonies.

As described above, in step (I-1), *Bacillus* bacteria capable of expressing PgsBCA and PgdS are used as the parent strain. In step (I-2), a *Bacillus* bacterium or a polynucleotide, or both, have a base sequence encoding a *Bacillus* bacteria-derived PgdS fragment, and/or in step (II), colonies are formed in the presence of *Bacillus* bacteria-derived PgdS protein. Thus, in step (II), colonies are formed in the presence of *Bacillus* bacteria-derived PgdS protein. The PgdS protein may be added (externally) to the plate medium. When added externally, the content of the PgdS protein in the medium is not limited but can be appropriately determined. Further, when the variant obtained in step (I) has a base sequence encoding a *Bacillus* bacteria-derived PgdS fragment, the variant strain may be cultured in the presence of a PgdS protein that the variant itself produces. Forming colonies in the presence of the PgdS protein in step (II) makes it possible to easily select a strain capable of forming colonies in spite of not lacking PgdS in step (III) described below.

Examples of the plate medium include, but are not limited to, known media capable of culturing *Bacillus* bacteria that produce γ-DL-PGA. Preferred examples include plate media, such as an LB agar medium (containing tryptone, yeast extract, sodium chloride, and agar, pH of 7). The culturing conditions are, for example, 30 to 37°C for 12 to 24 hours. Culturing is usually performed as a static culture, regardless of whether in light or darkness. Colonies are thereby formed on the medium.

As stated above, the variant strain obtained in step (I) may contain a base sequence encoding a genetic marker. In this case, from the viewpoint of more simply and selectively obtaining colonies that a variant forms, the plate medium is preferably, for example, a selective medium having a composition in accordance with the genetic marker. Although it is not intended to limit the present disclosure, when a drug resistance gene is used as a genetic marker and the drug resistance gene is a chloramphenicol gene, the selective medium preferably contains, for example, chloramphenicol as a drug. When the drug resistance gene is a kanamycin gene, the selective medium preferably contains, for example, kanamycin as a drug; and when the drug resistance gene is an erythromycin gene, the selective medium preferably contains, for example, erythromycin as a drug.

A preferred example of the selective medium is a medium obtained by mixing a known medium capable of culturing a *Bacillus* bacterium that produces γ-DL-PGA with a drug such as those mentioned above. Examples of the medium capable of culturing a known *Bacillus* bacterium that produces γ-DL-PGA include, but are not limited to, a plate medium, such as an LB agar medium. The content of the drug in the medium is not limited and may be appropriately determined according to the kind of drug and other factors. For example, the chloramphenicol content may be 5 to 10 µg/ml. The culture conditions are, for example, 30 to 37°C for 12 to 24 hours. Culturing is usually performed by static culture, regardless of whether in light or darkness. Colonies are thereby formed on the medium.

### Step (III)

From the colonies formed in step (II), colonies with luster are selected. Colonies with luster and/or colonies without luster may be present on the plate medium used in step (II). In step (III), colonies with luster are selected from the colonies formed on the plate medium.

"With luster" as referred to herein means that a water droplet-like luster is observed. When a known *Bacillus* bacterium capable of producing γ-DL-PGA is cultured on a plate medium that is generally used for culturing the *Bacillus* bacterium (e.g., LB plate medium), the bacterium forms colonies with water droplet-like luster to produce viscous γ-DL-PGA outside the cell. In selecting colonies with luster in step (III), while using as an indicator the luster of colonies observed when a known *Bacillus* bacterium that produces γ-DL-PGA is cultured, colonies having luster similar to this luster can be selected.

The known *Bacillus* bacterium capable of producing γ-DL-PGA as referred to herein means a strain that has the base sequence of SEQ ID NO: 2, the base sequence of SEQ ID NO: 4, the base sequence of SEQ ID NO: 6, and the base sequence of SEQ ID NO: 14, and whose culturing on an LB plate medium at 30 to 37°C for 12 to 24 hours results in expression and formation of a complex pgsBCAE composed of a PgsA protein encoded by the amino acid sequence of SEQ ID NO: 1, a PgsB protein encoded by the amino acid sequence of SEQ ID NO: 3, a PgsC protein encoded by the amino acid sequence of SEQ ID NO: 5, and a PgsE protein encoded by the amino acid sequence of SEQ ID NO: 13. A preferred strain is, for example, the strain obtained in Comparative Example 3 described below.

The colonies selected in step (III) are cultured and the average molecular weight of γ-L-PGA in the cultured product is determined to obtain a variant strain of *Bacillus* bacteria that produces ultra-high-molecular-weight γ-PGA with an average molecular weight of 20 million or more. The average molecular weight of γ-PGA is determined by measuring the weight average molecular weight in the same manner as described above. The medium used for the culturing is, for example, the above LB medium, MMOPS medium (1.5 g/l K₂HPO₄, 6 g/l NH₄Cl, 0.35 g/l MgSO₄·7H₂O, 0.05 g/l MnSO₄·5H₂O, 10.5 g/l 3-morpholinopropanesulfonic acid, 13.5 mg/l FeCl₃·6H₂0, 7.3 mg/l CaCl₂·2H₂O, 3.6 mg/l ZnSO₄·7H2O, 0.65 mg/l CuSO₄·5H₂O, 0.6 mg/l CoCl₂·6H₂O, and 0.6 mg/l Na₂MoO₄·2H₂O), MYC medium (MMOPS medium supplemented with 10 g/l glucose, 1 g/l Bacto yeast extract (Difco Laboratories, Franklin Lakes, NJ, USA), and 0.4 g/l casamino acids (Difco Laboratories)), and the like. For example, these media may or may not further contain a drug, such as those mentioned above, or other materials. The medium is a known medium used in PGA production. For example, MMOPS medium is a type of known minimal medium, and MYC medium is a medium having yeast extract and casamino acids added to MMOPS medium. Culture conditions are, for example, 30 to 37°C for 12 to 24 hours. Culturing is usually performed by static culture, in spite of whether in light or darkness.

When the average molecular weight determined in this manner is 20 million or more, it is determined that a variant strain of *Bacillus* bacteria that produces ultra-high-molecular-weight γ-PGA with an average molecular weight of 20 million or more has been obtained. On the other hand, when the average molecular weight determined in this manner is less than 20 million, it is determined that a variant strain of *Bacillus* bacteria that produces ultra-high-molecular-weight γ-PGA with an average molecular weight of 20 million or more has not been obtained. Thus, according to the screening method of the present disclosure, whether γ-PGA with an average molecular weight of 20 million or more has been produced can be easily determined, and whether a variant strain of *Bacillus* bacteria that produces ultra-high-molecular-weight γ-PGA with an average molecular weight of 20 million or more has been obtained can be easily determined. Thus, according to the screening method of the present disclosure, a variant strain of *Bacillus* bacteria that produces ultra-high-molecular-weight γ-PGA with an average molecular weight of 20 million or more can be easily screened for. This also means that according to the screening method of the present disclosure, a variant strain of *Bacillus* bacteria that produces ultra-high-molecular-weight γ-PGA with an average molecular weight of 20 million or more can be easily produced.

One of the purposes for which *Bacillus* bacteria produce γ-DL-PGA is adaptation to external stress. γ-DL-PGA is viscous. Efflux of viscous γ-DL-PGA around the cells is considered to protect cells from external stress. Colonies, if viscous, exhibit luster similar to water droplets. On the other hand, PgdS has the function of cleaving a bond between D-glutamic acid and L-glutamic acid, or a bond between L-glutamic acid and L-glutamic acid. When PgdS is overexpressed in a γ-DL-PGA-producing strain, the γ-DL-PGA discharged to outside of the cells is decomposed. Therefore, the colonies merely exhibit low viscosity and lose their luster. The present inventors focused on these properties and hypothesized that if *Bacillus* bacteria could acquire a mutation that produces γ-L-PGA in order to adapt themselves to external stress, thus avoiding the influence of PgdS, colonies would exhibit viscosity even under expression of PgdS. Based on this hypothesis, the present inventors attempted to screen for variant strains of *Bacillus subtilis* that produce γ-L-PGA. As a result, colonies that did not lose their luster even under the expression of PgdS appeared. In particular, the present inventors surprisingly found that ultra-high-molecular-weight γ-PGA with an average molecular weight of 20 million or more could be obtained. Thus, according to the present disclosure, ultra-high-molecular-weight γ-PGA with an average molecular weight of 20 million or more can be obtained, and a variant strain of *Bacillus* bacteria that produces ultra-high-molecular-weight γ-PGA with an average molecular weight of 20 million or more can be produced. The same explanation as above applies to the ultra-high-molecular-weight γ-PGA with an average molecular weight of 20 million or more. The present disclosure provides γ-PGA with an average molecular weight of 20 million or more, particularly γ-L-PGA with an average molecular weight of 20 million or more. The polyglutamic acid may or may not contain γ-D-glutamic acid.

Accordingly, for example, if necessary, the variant strain thus obtained may be further subjected to treatment to delete the ability to produce PgdS (for example, treatment to delete the base sequence encoding pgdS). As an example, since the ability to produce PgdS is not essential for the production of γ-PGA in the obtained variant strain, once a variant strain of *Bacillus* bacteria that produces γ-PGA with an average molecular weight of 20 million or more is obtained, the variant strain can still produce γ-PGA with an average molecular weight of 20 million or more even if the ability to produce PgdS is deleted from the variant strain.

### Variant strain of Bacillus bacteria that produces gamma-polyglutamic acid with an average molecular weight of 20 million or more

According to the screening method described above, a variant strain of *Bacillus* bacteria that produces γ-PGA with an average molecular weight of 20 million or more can be produced. Thus, the screening method described above can also be phrased as a method for producing a variant strain of *Bacillus* bacteria that produces γ-PGA with an average molecular weight of 20 million or more. Further, as is described below, the present inventors have succeeded in creating a variant strain of *Bacillus* bacteria that produces γ-PGA with an average molecular weight of 20 million or more.

Based on this success, the present disclosure provides a variant strain of *Bacillus* bacteria that produces ultra-high-molecular-weight γ-PGA with an average molecular weight of 20 million or more. The same explanation as above can apply to the ultra-high-molecular-weight γ-PGA with an average molecular weight of 20 million or more. The γ-L-PGA with an average molecular weight of 20 million or more may or may not contain γ-D-glutamic acid.

The variant strain of *Bacillus* bacteria that produces γ-PGA with an average molecular weight of 20 million or more according to the present disclosure preferably has a mutation in the sequence encoding PgsA among the 3 proteins that constitute the γ-PGA synthetase complex PgsBCA (or the 4 proteins that constitute the γ-PGA synthetase complex PgsBCAE).

The results of the Test Examples described below suggest that it is preferable that the variant strain of *Bacillus* bacteria that produces γ-L-PGA with an average molecular weight of 20 million or more is capable of expressing a PgsBCA complex (or PgsBCA) and it is also preferable that PgsA has membrane permeability and has a mutation that reduces or loses the activity of converting L-glutamic acid to D-glutamic acid. Specifically, it is suggested that in the cytoplasm, PgsB links L-glutamic acid to synthesize γ-L-PGA; γ-L-PGA is then transported to the outside of the cell by an export apparatus formed by the N-terminus of PgsC, N-terminus of PgsA, and C-terminus of PgsB, and that outside of the cell, PgsA converts part of the L-PGA to D-glutamic acid. Therefore, efflux of PGA to the outside of the cell does not occur in the presence of PgsB and PgsC (PgsBC) alone, thus failing to produce γ-L-PGA. Accordingly, it is considered necessary that in the variant strain, PgsA that constitutes the PgsBCA complex has at least the N-terminal region of PgsA (e.g., the amino acid sequence from position 1 to position 56 of SEQ ID NO: 1, which may be modified as described above), which is necessary for the efflux of PGA to the outside of the cell, and the variant strain must have a mutation that reduces or loses the activity of converting L-glutamic acid to D-glutamic acid. Considering this point, the variant of the present disclosure is preferably such that in a PgsBCA complex, the functions of PgsB and PgsC inherent in *Bacillus* bacteria that produce γ-DL-PGA are maintained, whereas PgsA has the N-terminal region of PgsA necessary for the efflux of γ-L-PGA to the outside of the cell and has a mutation that reduces or loses the activity of converting L-glutamic acid to D-glutamic acid outside of the cell. The same explanation also applies to the case in which the PgsBCA complex further comprises PgsE. In this case, the variant of the present disclosure preferably also maintains the function of PgsE inherent in *Bacillus* bacteria that produce γ-DL-PGA in the PgsBCA complex (PgsBCAE).

The variant strain can be produced by transformation of *a Bacillus* bacterium that produces γ-DL-PGA. The production method can be any method, such as natural mutation or introduction of a polynucleotide, and can be performed according to a conventional genetic engineering technique. A preferred example of the production method is production by performing the screening method described above.

As stated above, the variant strain is preferably capable of producing γ-L-PGA and having a mutation in the amino acid sequence or base sequence encoding PgsA. That is, the variant strain is preferably capable of expressing the complex PgsBCA (or PgsBCAE) and has a mutation in the amino acid sequence or base sequence encoding PgsA. Being capable of expressing the PgsBCA (or PgsBCAE) complex is not limited as long as the variant strain can express the complex when cultured on LB agar medium at 30°C for 30 hours. Further, examples of mutations in the amino acid sequence or base sequence encoding PgsA include, but are not limited to, the following mutations, as well as the mutations shown in Test Examples described below. The same explanation as above also applies to the amino acid sequences or base sequences encoding PgsB and PgsC, and the amino acid sequence or base sequence encoding PgsE, in the variant strain of the present disclosure. The same explanation as above also applies to modifications thereof etc. Further, as stated above, the variant strain may be capable of expressing PgdS. The same explanation as above also applies to the amino acid sequence or base sequence encoding PgdS, and the same explanation as above also applies to modifications thereof etc. Being capable of expressing PgdS is not limited as long as PgdS can be expressed when the variant strain is cultured on LB agar medium at 30°C for 30 hours.

Although not intended to limit the present disclosure, the following variant strains are mentioned as preferable examples of the variant strain of *Bacillus* bacteria that produces γ-PGA with an average molecular weight of 20 million or more.

Variant strains of *Bacillus subtilis* that satisfy the following features (A) and (B):
(A): PgsBCA is expressed;
(B): PgsA consists of any one of the following amino acid sequences (B1) to (B4):
   (B1): the amino acid sequence from position 1 to position 56 of the amino acid sequence of SEQ ID NO: 1;
   (B2): an amino acid sequence from position 1 to position 56 described above in (B1) in which one or more amino acids are deleted, substituted, or added;
   (B3): an amino acid sequence from position 1 to position 368 of the amino acid sequence of SEQ ID NO: 1 in which one or more amino acids are deleted, substituted, or added; and
   (B4) an amino acid sequence from position 1 to position 380 of the amino acid sequence of SEQ ID NO: 1 in which one or more amino acids are deleted, substituted, or added.

The same explanation as above applies to modifications, such as deletion, substitution, or addition, of one or more amino acids, in these amino acid sequences, as long as the number of modifications does not exceed the number of the amino acids. Further, the number of one or more amino acids modified in the amino acid sequence from positions 1 to 56 of the amino acid sequence of SEQ ID NO: 1, the amino acid sequence from positions 1 to 368 of the amino acid sequence of SEQ ID NO: 1, and the amino acid sequence from positions 1 to 380 of the amino acid sequence of SEQ ID NO: 1 is independently, for example, 1 to 30, preferably 1 to 25, 1 to 20, 1 to 15, 1 to 10, 1 to 8, 1 to 5, 1 to 4, 1 to 3, 1 or 2. The same explanation also applies to the variant strains described below.

Although not intended to limit the disclosure, the variant strain of *Bacillus subtilis* can be, for example, a variant strain of *Bacillus subtilis* that may have at least one of the following features (1) to (3):

(1) The amino acid sequences from position 1 to position 56 described above in (B2) to (B4) have at least one mutation selected from the group consisting of the following (1-1) to (1-5) :
   (1-1) F7S,
   (1-2) K20R,
   (1-3) V33A,
   (1-4) V35A, and
   (1-5) K50R.

For example, "F7S" means that F, which is the seventh amino acid residue in the amino acid sequence of SEQ ID NO:1, has been substituted with S. Similar explanations also apply to other descriptions. The same applies below. The one-letter codes for such amino acids follow the standard notation in this field.

(2) The amino acid sequence from positions 57 to 368 described above in (B3) and (B4) has at least one mutation selected from the group consisting of the following (2-1) to (2-98):
(2-1) D66V, (2-2) G70R, (2-3) F86V, (2-4) Q87*, (2-5) M69T, (2-6) R71R, (2-7) K75K, (2-8) Q79P, (2-9) K80E, (2-10) G81E, (2-11) E90K, (2-12) F93C, (2-13) F93L, (2-14) Y98Y, (2-15) N102S, (2-16) E104K, (2-17) E104V, (2-18) E104D, (2-19) E104G, (2-20) N105Y, (2-21) P106S, (2-22) P106L, (2-23) V107I, (2-24) N112D, (2-25) D117G, (2-26) K118E, (2-27) L122P, (2-28) L122Q, (2-29) T124P, (2-30) E127G, (2-31) K133R, (2-32) L140P, (2-33) N145S, (2-34) N145D, (2-35) H146Q, (2-36) H146R, (2-37) D149Y, (2-38) D149G, (2-39) D149N, (2-40) G151S, (2-41) V152V, (2-42) V152A, (2-43) Q153R, (2-44) T158R, (2-45) A163V, (2-46) F163S, (2-47) D168N, (2-48) G171V, (2-49) G171R, (2-50) A172V, (2-51) L176L, (2-52) S177R, (2-53) A179T, (2-54) K182A, (2-55) S184P, (2-56) K187R, (2-57) A194V, (2-58) G197D, (2-59) T199A, (2-60) A208T, (2-61) G214S, (2-62) L216L, (2-63) P217L, (2-64) M226T, (2-65) H233H, (2-66) H242Y, (2-67) H242R, (2-68) H242L, (2-69) E246E, (2-70) Y247H, (2-71) D250D, (2-72) M262V, (2-73) A265V, (2-74) V271A, (2-75) H274R, (2-76) P275L, (2-77) H276R, (2-78) H276Y, (2-79) L278L, (2-80) Y284H, (2-81) Y284C, (2-82) F298L, (2-83) D299G, (2-84) D299V, (2-85) D299G, (2-86) Q300R, (2-87) W302R, (2-88) L310Q, (2-89) L315P, (2-90) K316R, (2-91) K317E, (2-92) N318I, (2-93) G321E, (2-94) F323L, (2-95) T334A, (2-96) V338A, (2-97) T347A, (2-98) D355N.

In the above, Q87* means that the codon that encodes the amino acid residue at position 87 has become a stop codon (codon CAA that encodes Q at position 87 has been replaced with TAA).

(3) The amino acid sequence from positions 369 to 380 described above in (B4) has at least one mutation selected from the group consisting of the following (3-1) to (3-4) or deletion of (3-5):
(3-1) D370V,
(3-2) H373R,
(3-3) D375N,
(3-4) K376E,
(3-5) deletion of amino acid residues from position 369 to position 380.

In these modifications, the number of substitutions of amino acid residues at position 1 to position 380 of SEQ ID NO: 1 is preferably, for example, 1 to 30, and more preferably 1 to 20, 1 to 15, 1 to 10, 1 to 9, 1 to 8, 1 to 7, 1 to 6, 1 to 5, 1 to 4, 1 to 3, or 1 or 2.

The number of substitutions in amino acid residues from position 1 to position 56 of SEQ ID NO:1 is more preferably, for example, 0 to 7, and even more preferably, 0 to 6, 0 to 5, 0 to 4, 0 to 3, 0 to 2, 0 or 1.

The number of substitutions of amino acid residues from position 57 to position 368 of SEQ ID NO: 1 is more preferably, for example, 0 to 15, and even more preferably, 0 to 12, 1 to 10, 1 to 8, 1 to 5, 1 to 4, 1 to 3, 1 to 2, 1 or 2.

The number of substitutions of amino acid residues at positions 369 to 380 of SEQ ID NO: 1 is more preferably, for example, 0 to 8, and even more preferably 0 to 7, 0 to 6, 0 to 5, 0 to 4, 0 to 3, 0 to 2, 0 or 1.

The variant strain preferably, for example, has at least (2-4) Q87* mutation and/or (3-5) deletion of amino acid residues from position 369 to position 380.

The variant strain preferably, for example, has a mutation in at least one amino acid selected from the amino acids at positions 7, 93 to 122, 145 to 187, 213 to 250, 270 to 302, 310 to 323, and 330 to 338 of SEQ ID NO: 1. Among these, it is more preferable to have a mutation in at least one amino acid selected from the amino acids at positions 7, 93, 104, 122, 145, 146, 149, 152, 163, 171, 242, 247, 276, 299, 300, 317, and 338 of SEQ ID NO: 1.

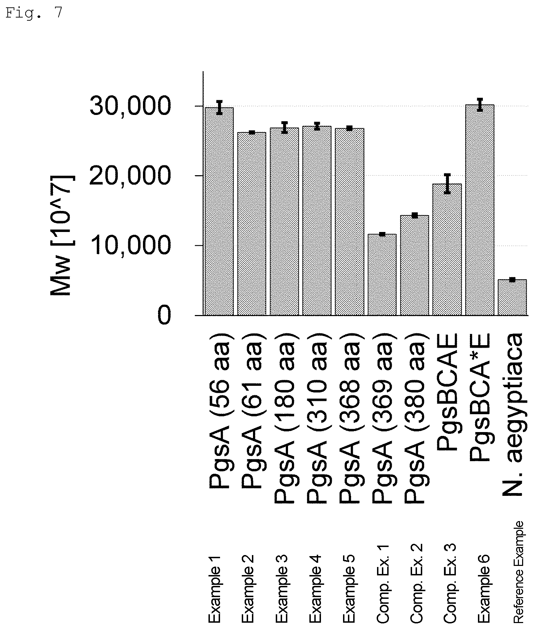

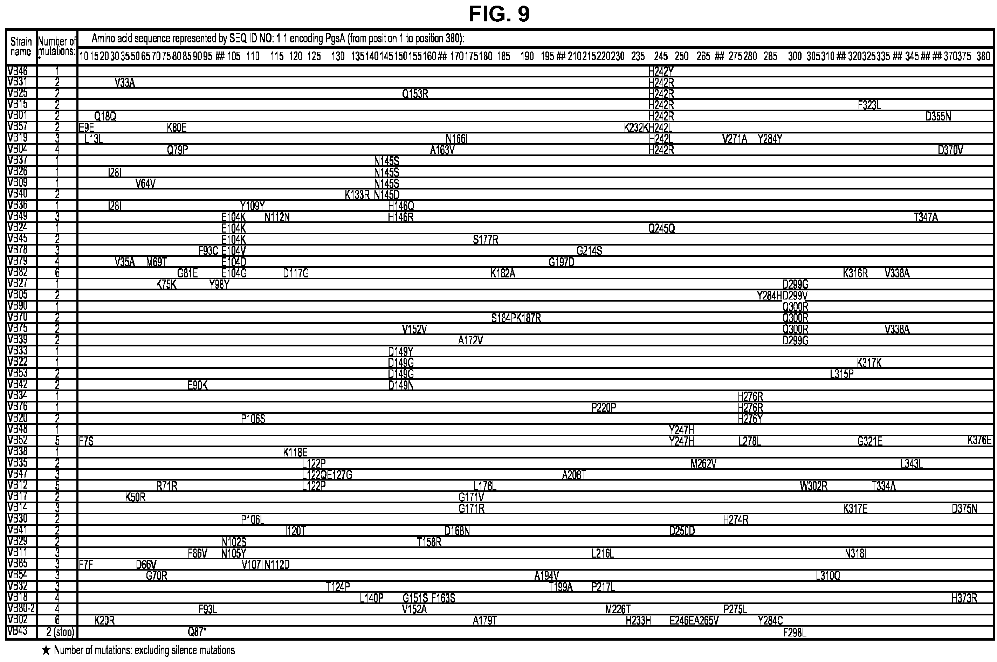

Examples of the variant strain of the present disclosure further include various variant strains obtained in the Test Examples described below (e.g., Figs. 7 and 9 and Tables 5 to 9).

The variant strain may have a silent mutation (a mutation in which the base sequence changes but the amino acid sequence does not change), and such a mutation may be any mutation as long as it is capable of producing γ-PGA with an average molecular weight of 20 million or more. Preferred examples include mutations obtained in the Test Examples described below (see, for example, Figs. 7 and 9 and Tables 5 to 9).

Variant strains other than *Bacillus subtilis,* such as *Bacillus amyloliquefaciens,* are also not limited, as stated above, and can be appropriately produced with reference to examples of modifications in *Bacillus subtilis.*

### Method for producing γ-polyglutamic acid with an average molecular weight of 20 million or more

From the above, the present disclosure can also be phrased to provide a method for producing γ-PGA with an average molecular weight of 20 million or more, which comprises the step of culturing a variant strain of *Bacillus* bacteria.

As described above, the present disclosure provides a variant strain of *Bacillus* bacteria that produces γ-PGA with an average molecular weight of 20 million or more. By culturing this variant strain, γ-PGA with an average molecular weight of 20 million or more can be produced.

The culturing is not limited as long as it is capable of culturing the variant strain of *Bacillus* bacteria of the present disclosure. Preferred examples of the culture medium include media that are generally used for culturing known *Bacillus* bacteria capable of producing γ-DL-PGA.

Examples of such media include LB medium, MMOPS medium, and MYC medium. Examples of culture conditions include 30 to 37°C for 12 to 48 hours. Culturing can be performed in light or in darkness, and can be performed according to a known procedure by any known culturing method, such as static culture or shaking culture. After culturing, γ-PGA having an average molecular weight of 20 million or more can be obtained from the obtained cultured product according to a conventional procedure known in the art, if necessary. The average molecular weight can be measured in the same manner as described above.

Thus, according to the present disclosure, γ-PGA having a very high average molecular weight of 20 million or more can be obtained. γ-PGA can be explained in the same manner as above.

Although γ-DL-PGA has been recognized as a promising bioplastic raw material, its practical implementation has advanced little. According to the present disclosure, ultra-high-molecular-weight γ-PGA with an average molecular weight of 20 million or more is obtained, and in particular, γ-L-PGA with an average molecular weight of 20 million or more is obtained. Further, although the archaeon *Natrialba aegyptiaca* is known as a microorganism capable of producing γ-L-PGA, culturing of the archaeon takes effort, for example, with it requiring rich nutrient sources, such as casamino acid. Further, the present inventors confirmed the molecular weight of γ-L-PGA produced by archaeon N. *aegyptiacaga,* which was found to be very low and merely 5085 kDa. In contrast, according to the present disclosure, ultra-high-molecular-weight γ-PGA, in particular γ-L-PGA, with an average molecular weight of 20 million or more can be provided, for example, by a general culture procedure. This is expected to lead to further advancements in the field of bioplastics.

### Examples

Embodiments of the present disclosure are described below more specifically with reference to examples. However, the embodiments of the present disclosure are not limited to the examples below.

### Test Example 1

### 1) Test Procedure

According to the following procedure, variant strains of *Bacillus* bacteria that produce γ-PGA with an average molecular weight of 20 million or more were prepared. Further, γ-PGA with an average molecular weight of 20 million or more was produced.

The primers used in the Test Examples are as shown in Table 1 below.

**Table 1**

| Primer name | Sequence | Template for PCR | SEQ ID No |
|---|---|---|---|
| ***rpsl-pgsA* (tet)** | | | |
| rpsl-1s | GGATTAAAATCAAGAACTGCTCTTG | MGB874 | 15 |
| rpsl-1f | GCTTCCAAAAGGTTCTCTAGGTCG | MGB874 | 16 |
| rpsl-1r-pgsA | CTTTTTTCATATTGGTAACCTCCTTTTTA TTAACGTTTTGAGAACTGAGGTGCAC | MGB874 | 17 |
| pgsA-f-SD | TAAAAAGGAGGTTACCAATATGAAAAAAG AACTGAGCTTTCATG | MGB874 | 18 |
| pgsA380-r | GCTCTTCTGGTGGAGTCTATCC CCGATTATTTAGATTTTAGTTTGTC | MGB874 | 19 |
| pgsA369-r | GCTCTTCTGGTGGAGTCTATCC TTA AAACGTCAGTTTTCCGTCTTCTAC | MGB874 | 20 |
| pgsA368-r | GCTCTTCTGGTGGAGTCTATCC TTA CGTCAGTTTTCCGTCTTCTACTTTC | MGB874 | 21 |
| pgsA310-r | GCTCTTCTGGTGGAGTCTATCC TTA CAGTGCACTGTCTCTTGTTCTCGTCC | MGB874 | 22 |
| pgsA180-r | GCTCTTCTGGTGGAGTCTATCC TTA TTTCGCATCACTTAAGCTGTATCCC | MGB874 | 23 |
| pgsA61-r | GCTCTTCTGGTGGAGTCTATCC TTA GGCTGAGAGTACGTCGTCAGAATACG | MGB874 | 24 |
| pgsA56-r | GCTCTTCTGGTGGAGTCTATCC TTA GTCAGAATACGTTTTGACCTTCGG | MGB874 | 25 |
| pgsA55-r | GCTCTTCTGGTGGAGTCTATCC TTA AGAATACGTTTTGACCTTCGGCGTTTC | MGB874 | 26 |
| pgsA48-r | GCTCTTCTGGTGGAGTCTATCC TTA CGGCGTTTCCGCTTTTCCCGCCCACATG | MGB874 | 27 |
| pgsA38-r | GCTCTTCTGGTGGAGTCTATCC TTA AGCGAACATAAGGACAAAAACGATCGG | MGB874 | 28 |
| rPCR-tetF | GGATAGACTCCACCAGAAGAGC CAGGTCGATATGAACAGC | pUC19 tet- | 29 |
| rPCR-tetR2 | CCAGGATGTAGTATCCTTCCG TCTCTCCCAAAGTTGATCCC | pUC19 tet- | 30 |
| rpsl-2f | CGGAAGGATACTACATCCTGG CTCAAAACGTTAATTTTACGTTTTC | MGB874 | 31 |
| rpsl-2r | CTTTGCTATTATGAGCTACTAACGC | MGB874 | 32 |
| rpsl-2s | GTTCGATAACCTGCTGTTCAACAGC | MGB874 | 33 |

| ***amyE*::P*_{rrnO}*-*pgsBCE (cat)*** | | | |
|---|---|---|---|
| amyE-UF | GGAGTGTCAAGAATGTTTGCAAAACGATTC | A-278 | 34 |
| amyE-DR2 | TCAATGGGGAAGAGAACCGCTTAAGCCCG | A-278 | 35 |
| DpgsA2-2f | GTGATAAAGATGAAATTTGTCAAAG | A-278 | 36 |
| DpgsA2-1r | CTTACATTAAATTAAGTAGTAAACAAAC | A-278 | 37 |

As the base sequences encoding pgsA, pgsB, pgsC, pgsS, and pgsE genes (fragments) described in the Test Example, the base sequences of SEQ ID NO: 2, SEQ ID NO: 4, SEQ ID NO: 6, SEQ ID NO: 8, and SEQ ID NO: 14 were used, respectively.

### 1-1) Production 1 of Bacillus subtilis (B. subtilis) variant strain

### - Production of complete pgsA gene-defective strain: Bacillus subtilis OA222 (MGB874:tufA-pgdS (spc), amyE::P_{rrnO}-pgsBCE (cat), ΔpgsBCAE-pgdS)

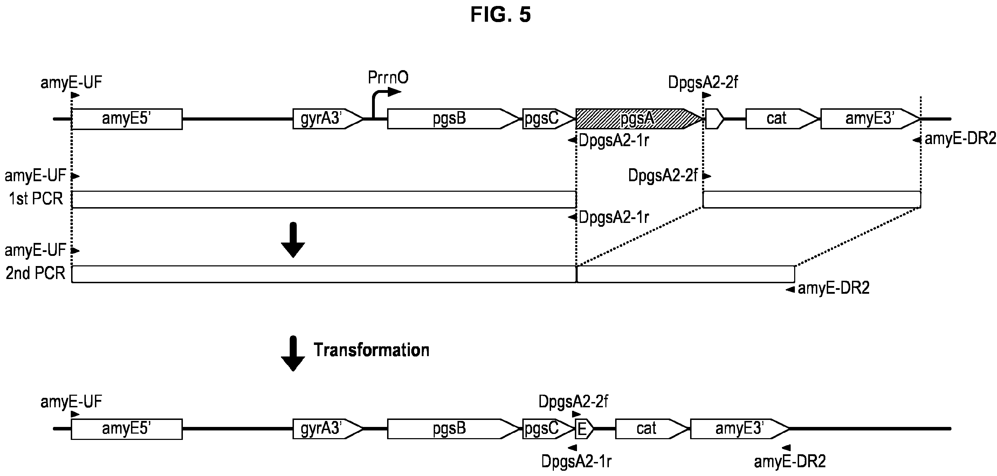

Using a *B. subtilis* A-278 (MGB874:tufA-pgdS(spc), amyE: :P_{rrnO}-pgsBCAE (cat), ΔpgsBCAE-pgdS) strain genome as a template, an upstream region not containing pgsA (from 5' end of amyE to pgsC) was amplified with amyE-UF/DpgsA2-1r, whereas a downstream region not containing pgsA (from pgsE to 3' end of amyE, including cat (chloramphenicol resistance gene)) was amplified with DpgsA2-2f/amyE-DR2. These fragments were ligated and amplified using amyE-UF/amyE-DR2 by OE (overlap extension)-PCR (Fig. 5).

In the process of producing the A-278 strain, a known strain from which the pgsBCAE-pgdS region had been deleted markerlessly and into which pgdS gene had been inserted as a transcriptional fusion downstream of tufA gene (MGB874:tufA-pgdS (spc), ΔpgsBCAE-pgdS) was used (Yamamoto J, et al. (2022), Constitutive expression of the global regulator AbrB restores the growth defect of a genome-reduced Bacillus subtilis strain and improves its metabolite production. DNA Res 29: dsac015). The fragment amplified by OE-PCR described above was introduced into this strain. After chloramphenicol-resistant colonies were selected on chloramphenicol-containing LB agar medium, a correct clone without mutations (having the fragment amplified by OE-PCR introduced thereinto) was designated as "OA222 strain". The OA222 strain can also be phrased as a parent strain in which pgdS gene (the base sequence encoding a pgdS fragment; the same applies below) has been inserted as a transcriptional fusion downstream of tufA gene and that also has pgsB gene, pgsC gene, pgsE gene, and a drug-resistant gene (cat).

### - Production of rpsI-pgsA(mut) strain

rpsI, which encodes a ribosomal protein, is an essential gene that is constitutively (always) transcribed at a high level. The present inventors produced strains in which wildtype or mutant pgsA gene was inserted as a transcriptional fusion downstream of rpsI.

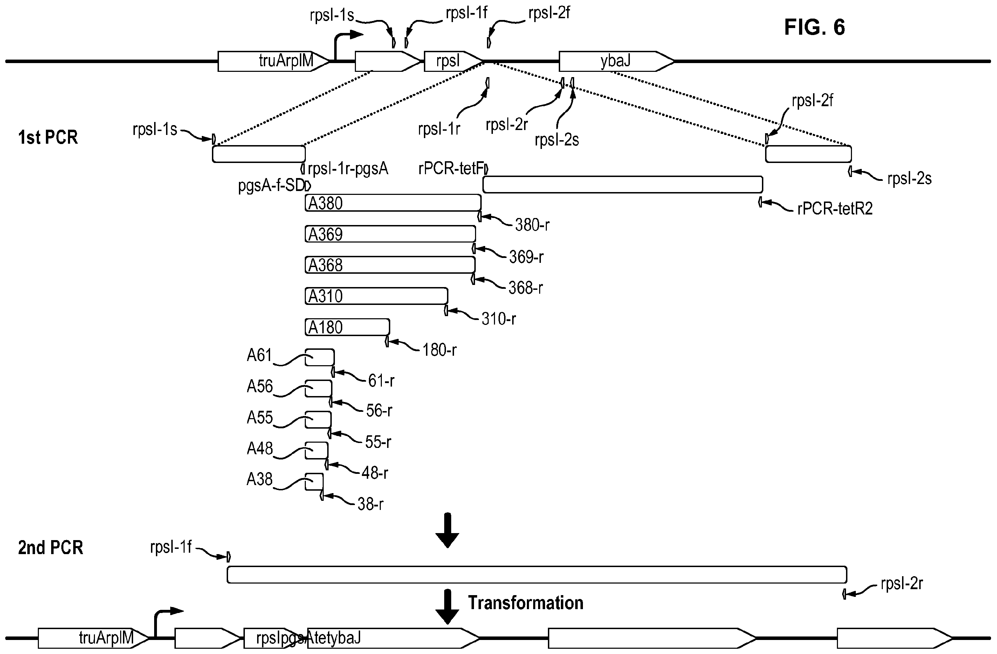

In the first PCR, using the *Bacillus subtilis* genome as a template, the upstream region of the insertion site was amplified with rpsI-1s/rpsI-1r-pgsA, and the downstream region was amplified with rpsI-2f/rpsI-2s. On the other hand, pgsA gene was amplified with pgsA-f-SD/pgsA380-r (or pgsA369-r, pgsA368-r, pgsA310-r, pgsA180-r, pgsA61-r, pgsA56-r, pgsA55-r, pgsA48-r, or pgsA38-r). A tetracycline-resistant gene cassette was amplified by rPCR-tetF/rPCR-tetR2 using pUC19 tet- as a template. These fragments were ligated by OE-PCR to obtain a polynucleotide (DNA fragment) to be introduced into the parent strain (Fig. 6). The DNA fragment contains rpsI gene and pgsA gene. Next, the DNA fragment was introduced into an OA222 strain. After tetracycline-resistant colonies were selected on tetracycline-containing LB agar medium, a correct clone without mutation (having pgsA gene introduced into the clone) (variant strain) was obtained.

### 1-2) Preparation 2 of Bacillus subtilis (B. subtilis) variant strain

In the process of transformation, natural variants capable of producing γ-L-PGA appeared frequently. Accordingly, first, a γ-L-PGA-producing strain was obtained by the transformation described below. The γ-DL-PGA-producing strain A-278, which overexpresses PgdS, was transformed with genomic DNA of strain OA193 (ΔkinA and PabrB(ΔSA)). Strain A-308 was obtained by selection with kanamycin and erythromycin. Further, strain A-327, which is a derivative strain of A-278 and is deficient in alsS-alsD (acetoin synthetic operon) was also transformed with genomic DNA of strain OA193 and selected with kanamycin to obtain strain OA203. After confirming that no mutations were present in pgdS gene, two clones having the features of L-PGA-producing strains were selected from each strain. The whole genome sequence was determined by using an Illumina sequencer, one of the nextgeneration sequencers, to identify mutations. The results show that all of the strains had mutations in pgsA (Table 2). Here, alsSD deficiency is known (Toya Y, et al. (2015), Enhanced dipicolinic acid production during the stationary phase in Bacillus subtilis by blocking acetoin synthesis. Biosci, Biotechnol, Biochem 79: 2073-2080), and kinA deficiency is also known.

**Table 2**

| Spontaneous *pgsA* mutations producing γ-L-PGA. | | | | |
|---|---|---|---|---|
| Strain | Transformation (antibiotics used for selection) | Mutation | Change in amino acid sequence | Other genotype |
| OA203-1- | OA193→ A-327 (kanamycin) | 1-bp substitution at 820-bp | His274Asp (C820G) | *ΔpgsBCAE-pgdS, tufA-pgdS (spc), amyE*::P*_{rrnO} -pgsBCAE (cat), ΔalsSD, ΔkinA (kan)* |
| OA203-2 | OA193→ A-327 (kanamycin) | 2-bp (GC) insertion at 515-bp | Amino acid changes from 172 aa and emergence of stop codon at 177th | *ΔpgsBCAE-pgdS, tufA-pgdS* (*spc*), *amyE*::*Pᵣᵣₙₒ -pgsBCAE (cat), ΔalsSD, ΔkinA (kan)* |
| A-308-FL4 | OA193→ A-278 (kanamycin & erythromycin) | 1-bp substitution | Asn145Asp (433G) | *ΔpgsBCAE-pgdS tufA-pgdS (spc), amyE*::*P_{rrnO} -pgsBCAE (cat), ΔkinA* (*kan*), *P_{abrB(ΔSA)}* (*erm*) |
| A-308-FL2 | OA193→ A-278 (kanamycin & erythromycin) | 24-bp (981-1004 bp) deletion | 328-335 aa deletion | *ΔpgsBCAE-pgdS, tufA-pqdS* (*spc*), *amyE*::*P_{rnnO}-pgsBCAE (cat), ΔkinA (kan), P_{abrB(ΔSA)}* (*erm*) |
| | | | | |
| A-278 | | | | *ΔpgsBCAE-pgdS, tufA-pgds (spc), amyE*::*P_{rrnO} -pgsBCAE (cat)* |
| A-327 | | | | *ΔpgsBCAE-pgdS tufA-pgdS* (*spc*), *amyE*::*P_{rrnO} -pgsBCAE (cat), ΔalsSD* |
| A-308 | | | | *ΔpgsBCAE-pgdS, tufA-pgdS* (*spc*), *amyE*::*P_{rrnO} -pgsBCAE (cat), ΔkinA (kan), P_{abrB(ΔSA)}* (*erm*) |
| OA193 | | | | *ΔkinA (kan),* P*_{abrB(ΔSA)}* (*erm*) |

Of the four obtained γ-L-PGA-producing variant strains having mutations in the pgsA gene, the strain OA203-1-L1 having pgsA* (His274Asp (His at position 274 in SEQ ID NO: 1 is mutated to Asp)) showed the highest viscosity derived from γ-PGA. Since this variant strain also had the mutations ΔalsSD and ΔkinA, pgsBCA*E gene was introduced into strain SA400N (MGB874 Trp+, ΔpgsBCAE, ΔpgdS). Specifically, genomic DNA (a DNA fragment) of strain OA203-1-L1 was introduced into strain SA400N, and chloramphenicol-resistant colonies were selected to create strain OA217 (MGB874 Trp+, ΔpgsBCAE, ΔpgdS, pgsBCA*E(cat)). The variant strain thus obtained is described as "PgsBCA*E" in Fig. 7 shown below. Strain SA400N can also be phrased as a parent strain deficient in pgdS gene, pgsB gene, pgsC gene, pgsA gene, and pgsE gene. The DNA fragment (polynucleotide) has pgdS gene, pgsB gene, pgsC gene, pgsA gene, and pgsE gene.

### 1-3) Production of γ-PGA and measurement of average molecular weight

The variant strain prepared as described above was inoculated onto LB agar medium and cultured at 30°C for 30 hours to form colonies. The obtained (viscous) colonies with luster were collected and suspended in distilled water. Cells were removed by centrifugation, and the resulting supernatant was filtered through a Cosmos Spin Filter H (0.45 µm; Nacalai Tesque, Kyoto, Japan). The resulting filtrate was eluted with 0.1 M Na₂SO₄ at 50°C and a flow rate of 1.0 mL/min. γ-L-PGA was detected at a wavelength of 210 nm using a UV detector. The molecular weight was analyzed using an HPLC device (LC-2030C Plus; Shimadzu Co., Ltd., Kyoto, Japan) equipped with size-exclusion chromatography (SEC) columns (TSKgel G6000PWXL and G4000PWXL; TOHSO, Tokyo, Japan). The γ-PGA content was calculated based on a calibration curve prepared using food-grade PGA (Meiji Food Material Co., Ltd., Tokyo, Japan). The molecular weight of γ-PGA was measured using a standard sample for size exclusion chromatography, pullulan STANDARD P-82 (Showa Denko K.K., Tokyo, Japan). The details are as follows:
Pullulan (Shodex STANDARD P-82; Showa, Tokyo, Japan) and 4 types of γ-PGA with known weight-average molecular weights (Mw), poly-L-glutamic acid sodium salts (molecular weight: 15000-50000; and
molecular weight: 50000-100000; Merck, Darmstadt, Germany), and poly-γ-glutamic acids (molecular weight: 200000-500000; and
molecular weight: 1500000-2500000; Fujifilm Wako Pure Chemical Corporation, Tokyo, Japan) were used as molecular-weight standards, and the weight-average molecular weight (Mw) of γ-L-PGA was determined using Shimadzu's LabSolutions GPC Software for HPLC.

As a reference example, the average molecular weight of γ-L-PGA produced by the archaeon *Natrialba aegyptiaca* was measured. The archaeon was cultured according to a known general procedure to form colonies. The average molecular weight of the obtained colonies was determined in the same manner as described above. The molecular weight of γ-L-PGA produced by the archaeon *N. aegyptiacaga* was very low, which was an average molecular weight of approximately 5090000 (5085 kDa).

### Results

Fig. 7 shows the results.

In Fig. 7, the variant strain "PgsA (56aa)" (Example 1) means a variant strain having, as PgsA, a protein encoded by the amino acid sequence from position 1 to position 56 of the amino acid sequence of SEQ ID NO: 1 encoding PgsA (on the N-terminal side). No difference was found between this variant strain and the parent strain in amino acid sequences encoding PgsB and PgsC. The γ-L-PGA produced by this variant strain had a weight-average molecular weight (Mw) of 29800000.

When the parent strain (PgsBCAE in Fig. 7) was cultured, the produced γ-L-PGA had a weight-average molecular weight (Mw) of 18850000 (18849 kDa). These results show that the ultra-high-molecular-weight γ-L-PGA produced by the variant strain PgsA (56aa) has a weight-average molecular weight (Mw) of 29.8 million, which is approximately 1.6 times higher than that of the parent strain.

Similarly, in Fig. 7, the variant strain "PgsA (61aa)" (Example 2) means a variant strain having, as PgsA, a protein encoded by the amino acid sequence from position 1 to position 61 of the amino acid sequence of SEQ ID NO: 1 encoding PgsA. No difference was also found between this variant strain and the parent strain in amino acid sequences encoding PgsB and PgsC. The γ-L-PGA produced by this variant strain had a weight-average molecular weight (Mw) of 26240000. This result shows that this variant strain also produces γ-L-PGA with a molecular weight of more than 20 million, which is higher than that of the parent strain.

A similar explanation also applies to the variant strain "PgsA (180aa)" (Example 3), variant strain "PgsA (310aa)" (Example 4), and variant strain "PgsA (368aa)" (Example 5) in Fig. 7.

Further, γ-L-PGA produced by the variant strain "PgsBCA*E" (Example 6) had a weight-average molecular weight (Mw) of 30200000 (30201 kDa). This result shows that this variant strain also produces γ-L-PGA with a molecular weight of more than 20 million, which is higher than that of the parent strain.

On the other hand, Fig. 7 shows that the variant strain "PgsA (369 aa)" (Comparative Example 1) and variant strain "PgsA (380 aa)" (Comparative Example 2) produced γ-L-PGA with a much higher molecular weight than *Bacillus subtilis* but that these variants did not have an average molecular weight of more than 20 million. The variant strain "PgsA (369aa)" is a variant strain having, as PgsA, a protein encoded by the amino acid sequence from position 1 to position 369 of the amino acid sequence of SEQ ID NO: 1 encoding PgsA. The variant strain "PgsA (380aa)" is a variant strain having, as PgsA, a protein encoded by the amino acid sequence from position 1 to position 380 of the amino acid sequence of SEQ ID NO: 1 encoding PgsA (i.e., the amino acid sequence of SEQ ID NO: 1). In contrast, the variant strain "PgsA (368aa)" is a variant having, as PgsA, a protein encoded by the amino acid sequence from position 1 to position 368 of the amino acid sequence of SEQ ID NO: 1 encoding PgsA. The above results show that the amino acid sequence from position 369 to position 380 of the amino acid sequence of SEQ ID NO: 1 as an amino acid sequence encoding PgsA is not essential for producing γ-L-PGA with an average molecular weight of more than 20 million, and that γ-L-PGA with an average molecular weight of more than 20 million is more likely to be obtained when the amino acid sequence from position 369 to position 380 is missing.

Although not shown in the results, colonies did not exhibit viscosity even when the amino acid sequence from position 1 to position 38 of SEQ ID NO: 1 was used as PgsA. Further, when the amino acid sequence from position 1 to position 48 or from position 1 to position 55 was used as PgsA, γ-L-PGA was not produced. These are sequences in which the transmembrane domain of PgsA is partially missing. On the other hand, as described above, when the amino acid sequence from position 1 to position 56 of SEQ ID NO: 1 was used as PgsA, γ-L-PGA with an average molecular weight of more than 20 million could be produced. This is a sequence maintaining the transmembrane domain of PgsA. These results indicate that the production of γ-L-PGA with a molecular weight of more than 20 million requires at least the N-terminal region containing the transmembrane region of PgsA in the PgsBCA (or PgsBCAE) complex.

Further, although not shown in the results, the ratio of γ-D-PGA to γ-L-PGA (D/L ratio) in strains having no mutations in PgsA, PgsB, PgsC, or PgsE was 68-69:31-32 (i.e., approximately 7:3), whereas the ratio in the variant strains PgsA (56aa), PgsA (61aa), PgsA (310aa), and PgsBCA*E described above was 0-2:98-100, the ratio in the variant strain PgsA (180aa) was 0-1:99-100, and the ratio in the variant strain PgsA (368aa) was 1-3:97-99, and a group of PGAs having a very high content ratio of γ-L-PGA to γ-D-PGA was obtained. These results reveal that such variants also have the feature of having a high ratio of γ-L-PGA to γ-D-PGA in the PGAs produced. In the calculation of this ratio, γ-L-PGA already proven to have an *N. aegyptoaca*-derived γ-L-PGA (Toyobo Co., Ltd.) ratio of 100% was used as a control and analyzed in the same manner. The analysis result showed that the ratio of γ-L-PGA to γ-D-PGA was 98% (as measured using a ligandexchange chiral column). These results show that the above variant strains, such as PgsA(56aa), were capable of efficiently producing γ-L-PGA rather than γ-DL-PGA. From the viewpoint of efficient production of γ-L-PGA, such variant strains are considered to be more preferable.

### Test Example 2

According to the following procedure, screening for a variant strain of *Bacillus* bacteria capable of producing γ-L-PGA having an average molecular weight of 20 million or more was carried out. The strains and primers used in this Test Example are as shown below.

**Table 3**

| **Strains** | |
|---|---|
| Strain | Genotype |
| OA-204-7 | MGB874 Trp+, *tufA*-bs*-pgdS*-spec, *amyE* :: P*rrnO* -bs(168)*-pgsBCAE -cat,* Δ*pgsBCAE, ΔpgdS ,* Δ*alsSD , PabrB* -Δ*spo0Abox* - erm |
| YU30-2 | MGB874 Trp+, *tufA -bs-pgdS -spec,* 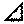 *pgsBCAE,* 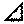 *pgdS* |
| YU31 | MGB874 Trp+, *tufA* -bs*-pgdS* -spec, 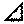 *pgsBCAE,* 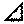 *pgdS, amyE*:: P*rrnO*-bs(168)-*pgsBCA***E* -cat |

| | |
|---|---|
| * pgsBCA*E: pgsBCAE having random mutation(s) introduced into pgsA: | |

**Table 4**

| **Primers** | | |
|---|---|---|
| Primer | Sequence | SEQ ID NO: |
| pgsA-1f | AATTTAATGTAAGGTGTGTCAAACG | 38 |
| pgsA-1r | ACAAATTTCATCTTTATCACTCCGA | 39 |
| pgsC-1r | CGTTTGACACACCTTACATTAAATTAAGTAGTAAACAAACATGATAGCAAAG | 40 |
| pgsE-1f | TCGGAGTGATAAAGATGAAATTTGTCAAAGCCATCTGGCCGTTTGTTGCC | 41 |
| amyE-1s | GGAGTGTCAAGAATGTTTGCAAAACGATTC | 42 |
| amyE-1f | ATTGCTGTTTCATTTGGTTCTGGCAGGACC | 43 |
| amyE-2s | CAATGGGGAAGAGAACCGCTTAAGCCCGAG | 44 |
| amyE-2r | ACGTAATCAAAGCCAGGCTGATTCTGACCG | 45 |

As base sequences encoding pgsA, pgsB, pgsC, pgdS, and pgsE genes (fragments) described in this Test Example, the same sequences as those in Test Example 1 were used.

### Testing Procedure

### Bacillus bacteria (parent strain)

As the parent strain, the *Bacillus subtilis* strain YU30-2 shown above in Table 3 was used. YU30-2 does not produce PGA because this strain lacks pgsBCDE gene and pgdS gene downstream of pgsBCDE gene, which are possessed by strains derived from *Bacillus subtilis* strain 168. However, since pgdS gene is inserted as a transcriptional fusion downstream of tufa and the downstream region is constantly transcribed at high levels, PgdS can be expressed at high levels.

### Polynucleotide (DNA Fragment)

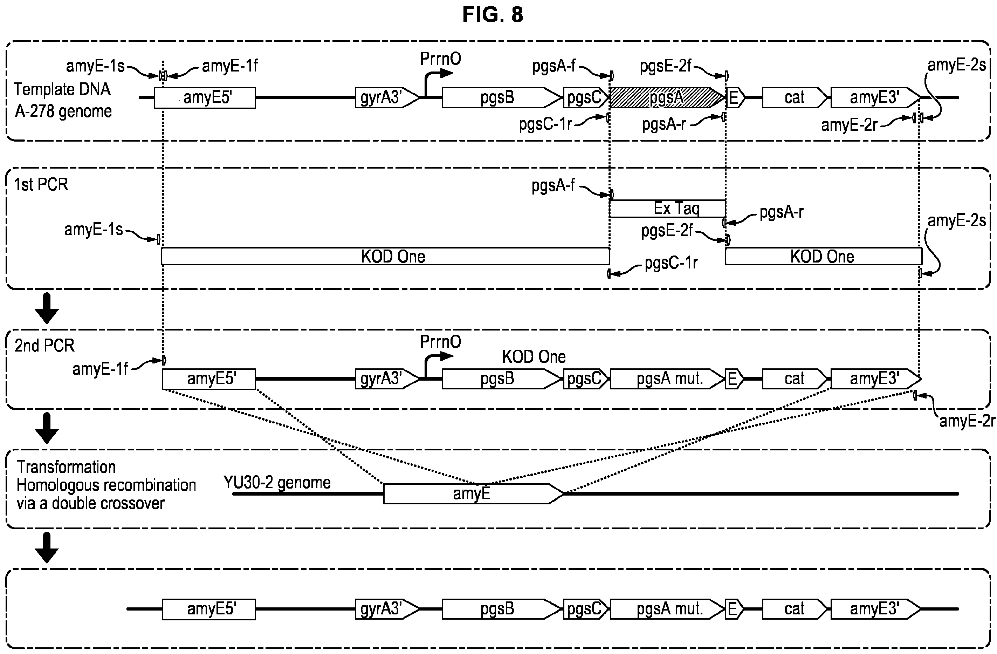

A DNA fragment was prepared in the following manner. In this Test Example, a drug-resistant gene was used as a genetic marker.
1) Using as a template the *Bacillus subtilis* strain A-278 having been confirmed to have no mutations in pgsBCAE, a PCR fragment was prepared. In the PCR using primer sets of amyE-1s/pgsC-1r and pgsE-1f/amyE-2s, KOD One (trademark) PCR Master Mix (TOYOBO) was used. For the pgsA region, PCR was performed using a primer set of pgsA-1f/pgsA-1r and using TaKaRa Ex Taq^{™} (Takara Bio Inc.).
2) The PCR fragments of each region were purified using Wizard^{™} SV Gel and PCR Clean-Up System (Promega), then ligated using NEBuilder^{™} HiFi DNA Assembly. A second PCR was performed using a primer set of amyE-1f/mmyE-2r and using KOD One^{™} PCR Master Mix (Toyobo Co., Ltd.). As a polynucleotide encoding a *Bacillus* bacteria-derived pgsBCAE fragment, the PCR product (DNA fragment) thus obtained was used for transformation. (Fig. 8)
3) The DNA concentration of the PCR fragment used in the transformation described below was 3.5 µg/µl.

### - Transformation

The parent strain described above was mixed with each PCR fragment and cultured in liquid medium to introduce the PCR fragment into the parent strain by natural transformation. Specifically, the PCR fragment was introduced in the following manner.

The parent strain was inoculated into 5 ml of CI medium to achieve an OD600 of 0.1 or less, and cultured with shaking at 37°C until the OD600 reached 1.5. The obtained culture medium was centrifuged (8000 rpm, 1 minute, room temperature (20°C)) to collect the cells. The supernatant was discarded, and the cells were suspended in 10 ml of C-II and cultured at 37°C for 40 minutes to obtain competent cells. To the culture medium thus obtained (1 ml of competent cells), 50 µl of the PCR product (DNA amount: 5 µg) was added and cultured at 37°C for 2 hours. The obtained cultured product was plated on selective medium. The selective medium used was LB agar medium (spectinomycin: 100 µg/ml; chloramphenicol: 7 µg/ml). The compositions of the CI medium and C-II medium used in this Test Example are as follows:

### Liquid A (/L)

K₂HPO₄ 28 g

KH₂PO₄ 12 g

(NH₄)₂SO₄ 4 g

Sodium citrate 2H₂O 2 g

### Liquid B (/L)

MgSO₄·7H₂O 2.5 g

D-glucose 10 g

0.1 mg/ml MnSO₄ 400 µl

2 mg/ml FeCl₃ 400 µl

100 x trace element (neither MnSO₄ nor FeCl₃ included) below 2 ml

### 100 x trace element (neither MnSO₄ nor FeCl₃ included) (/L)

CaCl₂ 550 mg

ZnCl₂ 170 mg

CuCl₂·2H₂O 43 mg

CoCl₂·6H₂O 60 mg

Na₂MO₄·2H₂O 60 mg

### C-I medium

Liquid A 2.5 ml

Liquid B 2.5 ml

5% yeast extract 50 µl

10% CAA 10 µl

5 mg/ml L-tryptophan 50 µl

### C-II medium

Liquid A 5 ml

Liquid B 5 ml

5% yeast extract 10 µl

10% casamino acids 10 µl

5 mg/ml L-tryptophan 10 µl

Strains that express PgdS can be selected by using 100 µg/ml spectinomycin. PgdS is an enzyme that hydrolyzes the D-Glu-D-Glu bond and the D-Glu-L-Glu bond in γ-PGA. Accordingly, the cells were statically cultured in selective medium at 37°C for 20 hours to form colonies. Among the colonies formed, colonies with luster were selected.

In the above experiment, 95 colonies with luster were collected. The number of colonies observed on the selective medium was 20 to 30 per plate. All the colonies with luster were viscous. The 95 colonies collected were numbered and designated YU31-VB01 to YU31-VB95. The obtained colonies were temporarily stored at -80°C and then inoculated into LB agar medium (spectinomycin: 100 µg/ml; chloramphenicol: 7 µg/ml) and cultured in the same manner as above. Since heterologous colonies were confirmed, single-colony isolation was performed and colonies with luster were selected.

Using the colonies thus obtained, pgsA mutations of the obtained variant strains were identified by Sanger sequencing according to known procedures.

### Results

Fig. 9 shows the results.

Further, mutations shown in Fig. 9 can be shown in Tables 5 to 9.

**Table 5**

| Stain name | Mutation site | | Mutant codon | | Mutant amino acid | |
|---|---|---|---|---|---|---|
| | bp | aa | Wild type | Mutation | Wild type | Mutation |
| YU31-VB01 | 54 | 18 | CAA | CAG | Q | Q |
| | 725 | 242 | CAC | CGC | H | R |
| | 1063 | 355 | GAC | AAC | D | N |
| YU31-VB02 | 59 | 20 | AAG | AGG | K | R |
| | 535 | 179 | GCG | ACG | A | T |
| | 699 | 233 | CAT | CAC | H | H |
| | 737 | 246 | GAG | GGG | E | E |
| | 794 | 265 | GCG | GTG | A | V |
| | 851 | 284 | TAT | TGT | Y | C |
| YU31-VB04 | 236 | 79 | CAA | CCA | Q | P |
| | 488 | 163 | GCG | GTG | A | V |
| | 725 | 242 | CAC | CGC | H | R |
| | 1109 | 370 | GAT | GGT | D | V |
| YU31-VB05 | 850 | 284 | TAT | CAT | Y | H |
| | 896 | 299 | GAC | GTC | D | V |
| YU31-VB09 | 192 | 64 | GTA | GTT | V | V |
| | 434 | 145 | AAC | AGC | N | S |
| YU31-VB10 | 208 | 70 | GGA | AGA | G | R |
| | 581 | 194 | GCG | GTG | A | V |
| | 929 | 310 | CTG | CAG | L | Q |
| YU31-VB11 | 256 | 86 | TTT | GTT | F | V |
| | 313 | 105 | AAC | TAC | N | Y |
| | 646 | 216 | CTG | TTG | L | L |
| | 954 | 318 | AAT | AAC | N | I |
| YU31-VB12 | 213 | 71 | CGC | CGT | R | R |
| | 365 | 122 | CTG | CCG | L | P |
| | 528 | 176 | TTA | TTG | L | L |
| | 904 | 302 | TGG | CGG | W | R |
| | 1000 | 334 | ACA | GCA | T | A |

**Table 6**

| Stain name | Mutation site | | Mutant codon | | Mutant amino acid | |
|---|---|---|---|---|---|---|
| | bp | aa | Wild type | Mutation | Wild type | Mutation |
| YU31-VB13 | 149 | 50 | AAG | AGG | K | R |
| | 512 | 171 | GGA | GTA | G | V |
| YU31-VB14 | 511 | 171 | GGA | AGA | G | R |
| | 949 | 317 | AAA | GAA | K | E |
| | 1123 | 375 | GAC | AAC | D | N |
| YU31-VB15 | 725 | 242 | CAC | CGC | H | R |
| | 967 | 323 | TTT | CTT | F | L |
| Y\U31-VB17 | 149 | 50 | AAG | AGG | K | R |
| | 512 | 171 | GGA | GTA | G | V |
| YU31-VB18 | 419 | 140 | CTC | CCC | L | P |
| | 451 | 151 | GGC | AGC | G | S |
| | 485 | 162 | TTT | TCT | F | S |
| | 1118 | 373 | CAT | CGT | H | R |
| YU31-VB19 | 37 | 13 | CTA | CTG | L | L |
| | 498 | 166 | AAT | AAC | N | I |
| | 725 | 242 | CAC | CTC | H | L |
| | 812 | 271 | GTC | GCC | V | A |
| | 852 | 284 | TAT | TAC | Y | Y |
| YU31-VB20 | 316 | 106 | CCG | TCG | P | S |
| | 826 | 276 | CAC | TAC | H | Y |
| YU31-VB22 | 446 | 149 | GAT | GGT | D | G |
| | 951 | 317 | AAA | AAG | K | K |
| YU31-VB23 | 310 | 104 | GAA | AAA | E | K |
| | 735 | 245 | CAA | CAG | Q | Q |
| YU31-VB24 | 310 | 104 | GAA | AAA | E | K |
| | 735 | 245 | CAA | CAG | Q | Q |
| YU31-VB25 | 458 | 153 | CAG | CGG | Q | R |
| | 725 | 242 | CAC | CGC | H | R |
| YU31-VB26 | 84 | 28 | ATT | ATC | I | I |
| | 434 | 145 | AAC | AGC | N | S |

**Table 7**

| Stain name | Mutation site | | Mutant codon | | Mutant amino acid | |
|---|---|---|---|---|---|---|
| | bp | aa | Wild type | Mutation | Wild type | Mutation |
| YU31-VB27 | 225 | 75 | AAA | AAG | K | K |
| | 294 | 98 | TAT | TAC | Y | Y |
| | 896 | 299 | GAC | GGC | D | G |
| YU31-VB28 | 899 | 300 | CAA | CGA | Q | R |
| YU31-VB29 | 305 | 102 | AAC | AGC | N | S |
| | 473 | 158 | ACG | AGG | T | R |
| YU31-VB30 | 317 | 106 | CCG | CTG | P | L |
| | 821 | 274 | CAC | CGC | H | R |
| YU31-VB31 | 98 | 33 | GTT | GCT | V | A |
| | 725 | 242 | CAC | CGC | H | R |
| YU31-VB32 | 370 | 124 | ACG | CCG | T | P |
| | 595 | 199 | ACC | GCC | T | A |
| | 650 | 217 | CCC | CTC | P | L |
| YU31-VB33 | 445 | 149 | GAT | TAT | D | Y |
| YU31-VB34 | 827 | 276 | CAC | CGC | H | R |
| YU31-VB35 | 365 | 122 | CTG | CCG | L | P |
| | 784 | 262 | ATG | GTG | M | V |
| | 1029 | 343 | CTT | CTC | L | L |
| YU31-VB36 | 84 | 28 | ATT | ATC | I | I |
| | 327 | 109 | TAT | TAC | Y | Y |
| | 438 | 146 | CAC | CAG | H | Q |
| YU31-VB37 | 434 | 145 | AAC | AGC | N | S |
| YU31-VB38 | 352 | 118 | AAA | GAA | K | E |
| YU31-VB39 | 515 | 172 | GCG | GTG | A | V |
| | 896 | 299 | GAC | GGC | D | G |
| YU31-VB40 | 398 | 133 | AAG | AGG | K | R |
| | 433 | 145 | AAC | GAC | N | D |
| YU31-VB41 | 359 | 120 | ATT | ACT | I | T |
| | 502 | 168 | GAT | AAT | D | N |
| | 750 | 250 | GAT | GAC | D | D |

**Table 8**

| Stain name | Mutation site | | Mutant codon | | Mutant amino acid | |
|---|---|---|---|---|---|---|
| | bp | aa | Wild type | Mutation | Wild type | Mutation |
| YU31-VB42 | 268 | 90 | GAA | AAA | E | K |
| | 445 | 149 | GAT | AAT | D | N |
| YU31-VB43 | 259 | 87 | CAA | TAA | Q | STOP |
| | 892 | 298 | TTT | CTT | F | L |
| YU31-VB45 | 310 | 104 | GAA | AAA | E | K |
| | 531 | 177 | AGT | AGA | S | R |
| YU31-VB46 | 724 | 242 | CAC | TAC | H | Y |
| YU31-VB47 | 365 | 122 | CTG | CAG | L | Q |
| | 380 | 127 | GAA | GGA | E | G |
| | 622 | 208 | GCT | ACT | A | T |
| YU31-VB48 | 739 | 247 | TAT | CAT | Y | H |
| YU31-VB48-2 | 739 | 247 | TAT | CAT | Y | H |
| YU31-VB49 | 310 | 104 | GAA | AAA | E | K |
| | 336 | 112 | AAT | AAC | N | N |
| | 437 | 146 | CAC | CGC | H | R |
| | 1039 | 347 | ACC | GCC | T | A |
| YU31-VB52 | 20 | 7 | TTT | TCT | F | S |
| | 739 | 247 | TAT | CAT | Y | H |
| | 832 | 278 | TTA | CTA | L | L |
| | 962 | 321 | GGA | GAA | G | E |
| | 1126 | 376 | AAA | GAA | K | E |
| YU31-VB53 | 446 | 149 | GAT | GGT | D | G |
| | 944 | 315 | CTG | CCG | L | P |
| YU31-VB54 | 208 | 10 | GGA | AGA | G | R |
| | 581 | 194 | GCG | GTG | A | V |
| | 929 | 310 | CTG | CAG | L | Q |
| YU31-VB57 | 27 | 9 | GAA | GAG | E | E |
| | 238 | 80 | AAA | GAA | K | E |
| | 696 | 232 | AAA | AAG | K | K |
| | 725 | 242 | CAC | CTC | H | L |

**Table 9**

| Stain name | Mutation site | | Mutant codon | | Mutant amino acid | |
|---|---|---|---|---|---|---|
| | bp | aa | Wild type | Mutation | Wild type | Mutation |
| YU31-VB65 | 21 | 7 | TTT | TTC | F | F |
| | 197 | 66 | GAT | GTT | D | |
| | 319 | 107 | GTA | ATA | V | V I |
| | 334 | 112 | AAT | GAT | N | D |
| YU31-VB70 | 550 | 184 | TCG | CCG | S | P |
| | 560 | 187 | AAA | AGA | K | R |
| | 899 | 300 | CAA | CGA | Q | R |
| YU31-VB75 | 456 | 152 | GTT | GTC | V | V |
| | 899 | 300 | CAA | CGA | Q | R |
| | 1013 | 338 | GTG | GCG | V | A |
| YU31-VB76 | 660 | 220 | CCT | CCC | P | P |
| | 827 | 276 | CAC | CGC | H | R |
| YU31-VB78 | 278 | 93 | TTT | TGT | F | C |
| | 311 | 104 | GAA | GTA | E | V |
| | 640 | 214 | GGC | AGC | G | S |
| YU31-VB79 | 104 | 35 | GTG | GCC | V | A |
| | 206 | 69 | ATG | ACG | M | T |
| | 312 | 104 | GAA | GAC | E | D |
| | 590 | 197 | GGC | GAC | G | D |
| YU31-VB80 | 277 | 93 | TTT | CTT | F | L |
| | 455 | 152 | GTT | GCT | V | A |
| | 677 | 226 | ATG | ACG | M | T |
| | 824 | 275 | CCG | CTG | P | L |
| YU31-VB80-2 | 277 | 93 | TTT | CTT | F | L |
| | 455 | 152 | GTT | GCT | V | A |
| | 677 | 226 | ATG | ACG | M | T |
| | 824 | 275 | CCG | CTG | P | L |
| YU31-VB82 | 242 | 81 | GGG | GAG | G | E |
| | 311 | 104 | GAA | GGA | E | G |
| | 350 | 117 | GAT | GGT | D | G |
| | 544 | 182 | AAA | GAA | K | A |
| | 947 | 316 | AAG | AGG | K | R |
| | 1013 | 338 | GTG | GCG | V | A |
| YU31-VB90 | 899 | 300 | CAA | CGA | Q | R |

As shown in Fig. 9 and Tables 5 to 9, many variant strains having mutations in the amino acid sequence or base sequence encoding PgsA in complex proteins constituting pgsBCAE could be prepared. Since mutations were introduced randomly into pgsA in this Test Example, many variant strains with mutations in the amino acid sequence and the base sequence encoding PgsA were expected to be obtained among the variant strains obtained. As expected, the results show that many variant strains having mutations in the amino acid sequence or base sequence encoding PgsA could be obtained. Thus, according to this method, a desired variant strain can be easily screened for and can also be easily produced. Further, the production of γ-PGA, and more specifically γ-L-PGA, with an average molecular weight of 20 million or more can be confirmed by measuring the average molecular weight of γ-L-PGA produced by the obtained variant strain in the same manner as described above.

It was found from the above that a variant strain of a *Bacillus* bacterium capable of producing γ-L-PGA with an average molecular weight of 20 million or more can be produced by introducing a mutation of reducing or losing the activity of converting L-glutamic acid to D-glutamic acid into a *Bacillus* bacterium capable of producing γ-PGA in such a manner that the obtained variant strain of the *Bacillus* bacterium is capable of expressing the γ-PGA synthase complex PgsBCA (or PgsBCAE) and PgsA has membrane permeability. It was also found that since such a variant strain of a *Bacillus* bacterium can be easily prepared, γ-L-PGA with an average molecular weight of 20 million or more can also be easily produced according to the present disclosure.

## Claims

1. A γ-polyglutamic acid with an average molecular weight of 20 million or more.

2. A screening method for a variant strain of *Bacillus* bacteria that produces a γ-polyglutamic acid with an average molecular weight of 20 million or more, the method comprising the following steps (I) to (III):
(I) the following step (I-1) or (I-2)
(I-1) the step of culturing a *Bacillus* bacterium capable of expressing PgsBCA and PgdS in a medium, and
(I-2) the step of mixing a *Bacillus* bacterium with a polynucleotide and culturing the mixture in a medium,
(II) the step of inoculating the cultured product obtained in step (I) onto a plate medium to allow colonies to form, and
(III) the step of selecting colonies with luster from the colonies formed in step (II),
wherein in step (I-2),
the *Bacillus* bacterium or the polynucleotide, or both, have a base sequence encoding a *Bacillus* bacteria-derived pgsA fragment,
the *Bacillus* bacterium or the polynucleotide, or both, have a base sequence encoding a *Bacillus* bacteria-derived pgsB fragment,
the *Bacillus* bacterium or the polynucleotide, or both, have a base sequence encoding a *Bacillus* bacteria-derived pgsC fragment, and,
the *Bacillus* bacterium or the polynucleotide, or both, have a base sequence encoding a *Bacillus* bacteria-derived PgdS fragment, and/or in step (II), colonies are allowed to form in the presence of *Bacillus* bacteria-derived PgdS protein.

3. The screening method according to claim 2, further comprising the step of culturing the colonies selected in step (III) and determining the average molecular weight of a γ-polyglutamic acid in the cultured product.

4. The screening method according to claim 2 or 3, wherein the *Bacillus* bacterium is *Bacillus subtilis.*

5. A method for producing a γ-polyglutamic acid with an average molecular weight of 20 million or more,
the method comprising the step of culturing the variant strain of *Bacillus* bacteria obtained according to the screening method of claim 2 or 3.

6. A variant strain of *Bacillus* bacteria that produces a γ-polyglutamic acid with an average molecular weight of 20 million or more.

7. The variant strain of *Bacillus* bacteria according to claim 6, wherein the variant strain of *Bacillus* bacteria is a variant strain of *Bacillus subtilis* that satisfies the following (A) and (B):
(A) the variant strain expresses PgsBCA, and
(B) PgsA consists of the amino acid sequence of any one of the following (B1) to (B4):
(B1) the amino acid sequence from position 1 to position 56 of the amino acid sequence of SEQ ID NO: 1,
(B2) an amino acid sequence in which one or more amino acids are deleted, substituted, or added in the amino acid sequence from position 1 to position 56 described in (B1),
(B3) an amino acid sequence in which one or more amino acids are deleted, substituted, or added in the amino acid sequence from position 1 to position 368 of the amino acid sequence of SEQ ID NO: 1, and
(B4) an amino acid sequence in which one or more amino acids are deleted, substituted, or added in the amino acid sequence from position 1 to position 380 of the amino acid sequence of SEQ ID NO: 1.
